# EUROPEAN PATENT APPLICATION

(11) **EP 2 574 346 A1**
(43) Date of publication of application: **03.04.2013**
(21) Application number: 12191778.5
(22) Date of filing: 24.03.2004
(51) Int. Cl.: A61K 39/395, C07K 16/28, A61K 38/21, A61K 39/20, A61K 38/19, A61K 38/20

(54) **Cytokine-expressing cellular vaccine combinations**

(30) Priority: 02.04.2003 US 404662
(62) Divisional of application: 04759735.6
(71) Applicant: BioSante Pharmaceuticals, Inc., Lincolnshire, IL 60069 (US)
(72) Inventor: Jooss, Karin, Bellevue, WA 98005 (US); Creson, Jennifer, San Mateo, CA 94401 (US); Li, Betty, San Francisco, CA 94132 (US); Prell, Rodney, Richmond, CA 94804 (US); Aung, Sandra, San Francisco, CA 94115 (US); Moskalenko, Marina, Pacifica, CA 94044 (US); Du, Thomas, San Bruno, CA 94066 (US)
(74) Representative: HOFFMANN EITLE

(57) **Abstract**

The present invention in all of its associated aspects provides improved methods and compositions for treating cancer in a mammal based on the administration of the combination of a cytokine-expressing cellular vaccine and at least one additional cancer therapeutic agent or treatment to a patient with cancer, wherein administration of the combination results in enhanced therapeutic efficacy relative to administration of the cytokine-expressing cellular vaccine or cancer therapeutic agent or treatment as a monotherapy.

## Description

This application is a continuation-in-part of U.S. Patent Application Serial No. 10/404,662 which was filed on April 2, 2003. The entirety of that application is incorporated herein by reference.

### Field of The Invention

The present invention relates to a method of altering an individual's immune response to a target cancer antigen or antigens. More particularly, the invention is concerned with compositions comprising a cytokine-expressing cellular vaccine and at least one additional cancer therapeutic agent or treatment and methods of administering a cytokine-expressing cellular vaccine combination comprising a cytokine-expressing cellular vaccine and at least one additional cancer therapeutic agent or treatment to a patient with cancer.

### Background Of The Invention

The immune system plays a critical role in the pathogenesis of a wide variety of cancers. When cancers progress, it is widely believed that the immune system either fails to respond sufficiently or fails to respond appropriately, allowing cancer cells to grow. Currently, standard medical treatments for cancer including chemotherapy, surgery, radiation therapy and cellular therapy have clear limitations with regard to both efficacy and toxicity. To date, these approaches have met with varying degrees of success dependent upon the type of cancer, general health of the patient, stage of disease at the time of diagnosis, etc. Improved strategies that combine specific manipulation of the immune response to cancer in combination with standard medical treatments may provide a means for enhanced efficacy and decreased toxicity.

The use of autologous cancer cells as vaccines to augment anti-tumor immunity has been explored for some time (Oettgen et al., "The History of Cancer Immunotherapy", In: Biologic Therapy of Cancer, Devita et al. (eds.) J. Lippincot Co., pp87-199, 1991). However, due to the weak immunogenicity of many cancers, down regulation of MHC molecules, the lack of adequate costimulatory molecule expression and secretion of immunoinhibitory cytokines by cancer cells, the response to such vaccines has not resulted in long term efficacy. See, e.g., Armstrong TD and Jaffee EM, Surg Oncol Clin N Am. 11(3):681-96, 2002 and Bodey B et al., Anticancer Res 20(4):2665-76, 2000.

Numerous cytokines have been shown to play a role in regulation of the immune response to tumors. For example, U.S. Patent No. 5,098,702 describes using combinations of TNF, IL-2 and IFN-beta in synergistically effective amounts to combat existing tumors. U.S. Patent Nos. 5,078,996, 5,637,483 and 5,904,920 describe the use of GM-CSF for treatment of tumors. However, direct administration of cytokines for cancer therapy may not be practical, as they are often systemically toxic. (See, for example, Asher et al., J. Immunol. 146: 3227-3234, 1991 and Havell et al., J. Exp. Med. 167: 1067-1085, 1988.)

An expansion of this approach involves the use of genetically modified tumor cells which express cytokines locally at the vaccine site. Activity has been demonstrated in tumor models using a variety of immunomodulatory cytokines, including IL-4, IL-2, TNF-alpha, G-CSF, IL-7, IL-6 and GM-CSF, as described in Golumbeck PT et al., Science 254:13-716,1991; Gansbacher B et al., J. Exp. Med. 172:1217-1224, 1990; Fearon ER et al., Cell 60:397-403, 1990; Gansbacher B et al., Cancer Res. 50:7820-25, 1990; Teng M et al., PNAS 88:3535-3539,1991; Columbo MP et al., J. Exp. Med. 174:1291-1298, 1991; Aoki et al., Proc Natl Acad Sci U S A. 89(9):3850-4,1992; Porgador A, et al., Nat Immun. 13(2-3):113-30, 1994; Dranoff G et al., PNAS 90:3539-3543, 1993; Lee CT et al., Human Gene Therapy 8:187-193, 1997; Nagai E et al., Cancer Immunol. Immonther. 47:2-80,1998 and Chang A et al., Human Gene Therapy 11:839-850, 2000, respectively.

Clinical trials employing GM-CSF-expressing autologous or allogeneic cellular vaccines (GVAX®) have commenced for treatment of prostate cancer, melanoma, lung cancer, pancreatic cancer, renal cancer, and multiple myeloma (Dummer R., Curr Opin Investig Drugs 2(6):844-8, 2001; Simons J et al., Cancer Res. 15;59(20):5160-8, 1999; Soiffer R et al., PNAS 95:13141-13146, 1998; Simons J et al., Cancer Res. 15; 57:1537-1546, 1997; Jaffee E et al., J. Clin Oncol. 19:145-156, 2001; and Salgia R et al., J. Clin Oncol. 21:624-630, 2003).

In yet another approach, autologous tumor cells were genetically altered to produce a costimulatory molecule, such as B7-1 or allogeneic histocompatibility antigens (Salvadori et al. Hum. Gene Ther. 6:1299-1306, 1995 and Plaksin et al. Int. J. Cancer 59:796-801,1994). While the use of genetically modified tumor cells has met with success in treatment of some forms of cancer, there remains a need for improved treatment regimens with greater potency/efficacy and less side effects than the therapies currently in use.

### Summary Of The Invention

The invention provides improved compositions and methods for the treatment of cancer in a mammal, typically a human, by administering a cytokine-expressing cellular vaccine combination comprising a cytokine-expressing cellular vaccine and at least one additional cancer therapeutic agent to a subject with cancer.

Administration of a cytokine-expressing cellular vaccine combination results in enhanced therapeutic efficacy and/or vaccine potency relative to administration of the cytokine-expressing cellular vaccine or the additional cancer therapeutic agent alone.

In one aspect of the invention, the cytokine expressing cellular vaccine expresses GM-CSF.

In another aspect of the invention, the cytokine-expressing cellular vaccine combination comprises cells that are autologous, allogeneic or bystander cells.

In a further aspect of the invention, a population of autologous, allogeneic or bystander cells are genetically modified to produce an effective amount of a cytokine, e.g. GM-CSF.

The at least one additional cancer therapeutic agent is selected from the group consisting of an anti-CTLA4 antibody, an anti-4-1BB antibody, interferon-alpha, docetaxel, paclitaxel, a COX-2 inhibitor, an anti- CD40 antibody or CD40 ligand expressed on the cell surface of the tumor cell vaccine, an anti-OX40 antibody or OX-40 ligand expressed on the cell surface of the tumor cell vaccine, a CpC oligonucleotide and a heat shock protein (HSP).

The same or a different population of autologous, allogeneic or bystander cells may be genetically modified to produce an effective amount of at least one additional cancer therapeutic agent.

The autologous, allogeneic or bystander cells are rendered proliferation incompetent by irradiation prior to administration to the subject.

In yet a further aspect of the invention, the at least one additional cancer therapeutic agent is administered as a chemical entity such as an antibody (e.g., an anti-CTLA4 antibody, an anti-4-1BB antibody, an anti-CD40 antibody and an anti-OX40 antibody), as traditional chemotherapy, e.g., docetaxel, paclitaxel or as a cell-surface molecule expressed on the vaccine cells.

The cytokine-expressing cellular vaccine is typically administered subcutaneously or intratumorally. The at least one additional cancer therapeutic agent may be administered prior to, at the same time as, or following administration of the cytokine-expressing cellular vaccine component of the combination.

The invention further provides compositions and kits comprising cytokine-expressing cellular vaccine combinations for use according to the description provided herein.

### Brief Description Of The Drawings

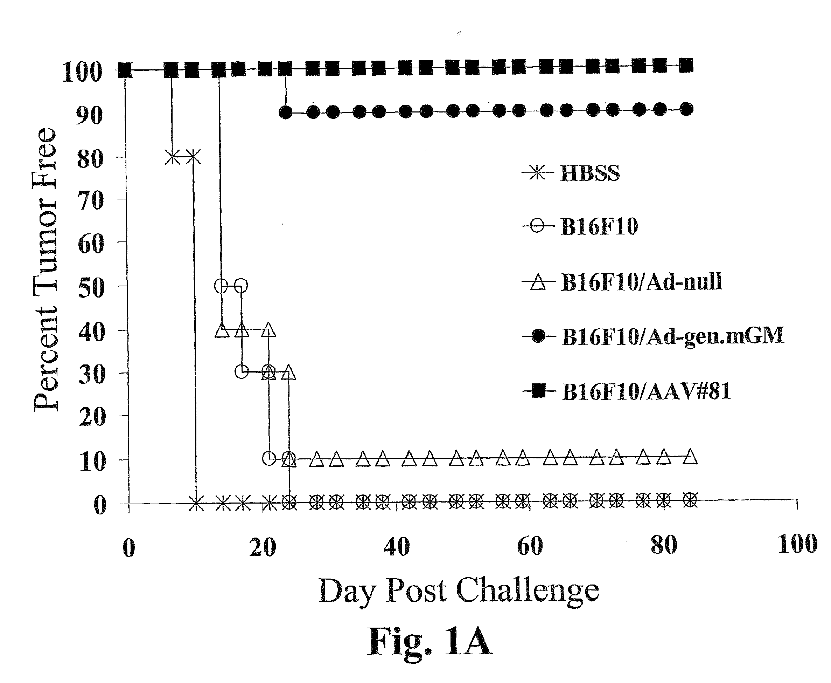

Figure 1A is a graphic depiction of the results of a study in C57BI/6 mice indicating tumor free survival following injection of a 6M-CSF-secreting tumor cell vaccine in a basic prevention model. In this model, mice were vaccinated (subcutaneously; SC) with 1x10⁶ irradiated tumor cells. Seven days later, mice were challenged (SC) with 1x10⁶ B16F10 tumor cells and followed for tumor development and survival. B16F10/Ad-null is a B16F10 clone transduced with an E1-deleted adenoviral vector expressing no transgene B16F10/Ad-gen.mGM is a B16F10 clone transduced with an E1-deleted adenoviral vector expressing 450 ng/1x10⁶ cells/24h of 6M-CSF from genomic DNA. AAV#81 is a B16F10 clone that was transduced with adenovirus expressing GM-CSF. AAV#81 produced an average of 500ng GM-CSF/24h/1x10⁶ cells.

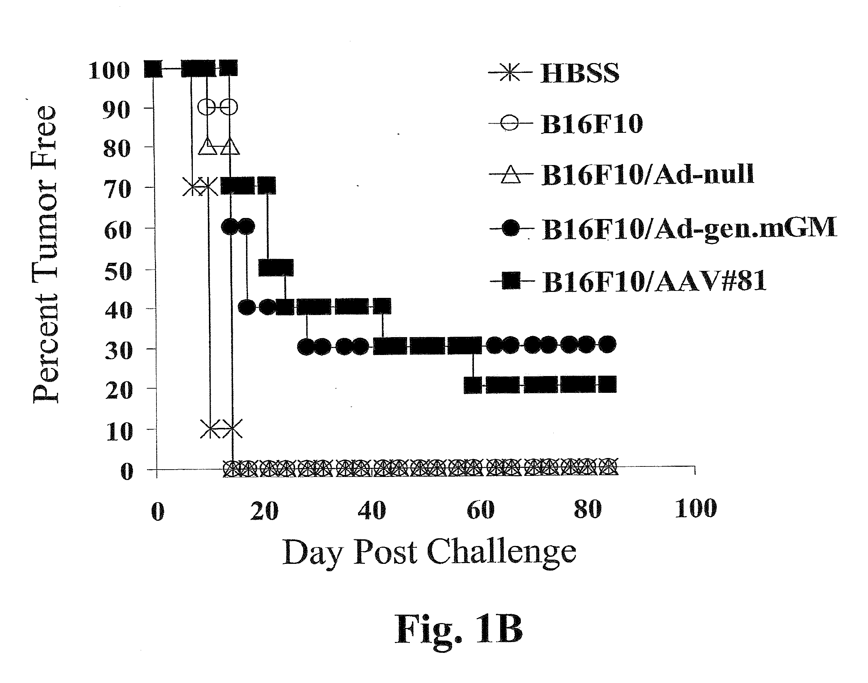

Figure 1B is a graphic depiction of the results of a study in C57BI/6 mice indicating tumor free survival following injection of a GM-CSF-secreting tumor cell vaccine in a treatment model. In this model mice were challenged (SC) with 5x10⁴ B16F10 tumor cells and 3 days later treated with 1x10⁶ irradiated tumor cells. Mice were followed for tumor development and survival. B16F10/Ad-null is a B16F10 clone transduced with an E1-deleted adenoviral vector expressing no transgene B16F10/Ad-gen.mGM is a B16F10 clone transduced with an E1-deleted adenoviral vector expressing 450 ng/1x10⁶ cells/24h of GM-CSF from genomic DNA. AAV#81 is a B16F10 clone that was transduced with adenovirus expressing GM-CSF. AAV#81 produced an average of 500ng GM-CSF/24h/1x10⁶ cells.

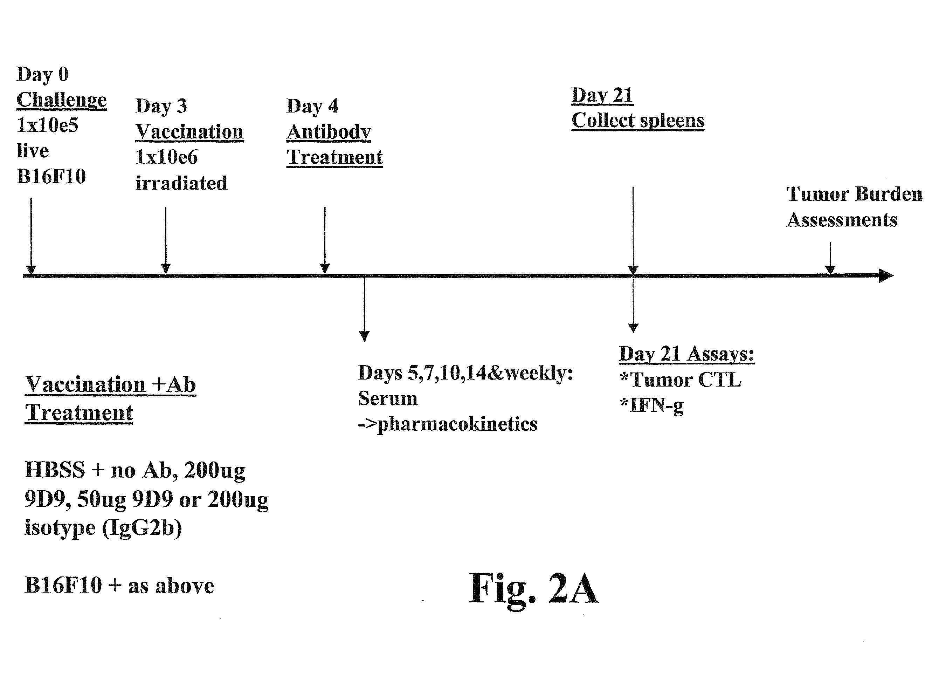

Figure 2A is a schematic depiction of a study in the B16F10 melanoma model where C57BI6 mice were challenged with 1x10⁵ B16F10 tumor cells on Day 0. One day later, mice were vaccinated with 1x10⁶ irradiated B16F10 or GM-CSF-secreting B16F10 cells. One and three days after vaccination, mice were injected with 150ug and 100ug of anti-CTLA-4 antibody (9D9 is a mouse anti mouse anti-CTLA4 ab or 9H10 which is a hamster anti mouse anti-CTLA4 ab), respectively. In some cases, mice received a second vaccination of 3x10⁶ irradiated cells two days after the last anti-CTLA-4 treatment. Mice were then monitored for the development of subcutaneous tumors, subjected to immunological monitoring and sacrificed on Day 21.

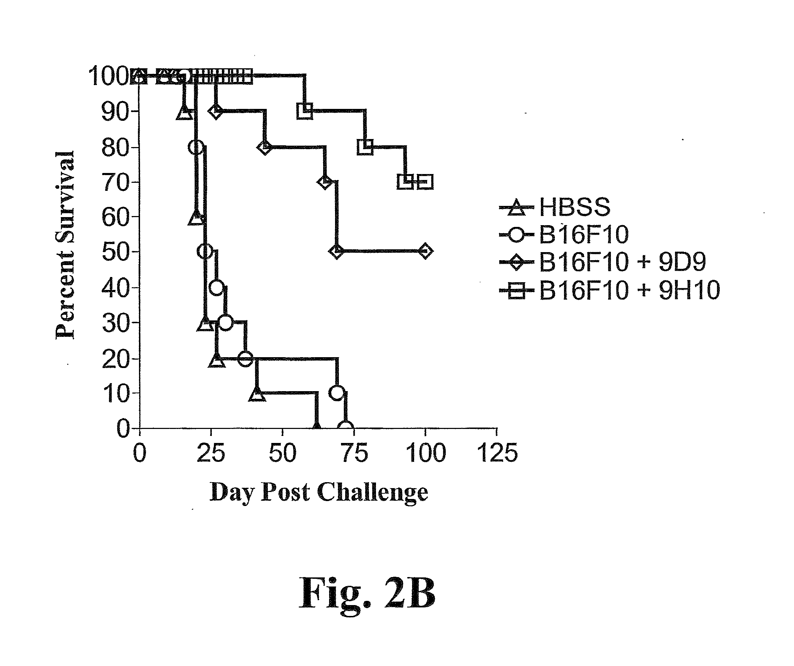

Figure 2B illustrates the results of treatment with the combination of B16F10 or GM-CSF-secreting B16F10 (dB16gmtd) cells and a hamster (9H10) or murine (9D9) anti-CTLA-4 antibody in the B16F10 model using a regiment similar to that depicted in Fig. 2A. C57BI6 mice were challenged with 1x10⁵ B16F10 tumor cells. One day later, mice were vaccinated with 3x10⁶ irradiated B16F10 or GM-CSF-secreting B16F10 (dB16gmtd) cells. One and three days after vaccination, mice were injected with 150ug and 100ug of anti-CTLA-4 (9D9 or 9H10; Fig 2A), respectively. Mice received a second vaccination of 3x10⁶ irradiated cells two days after the last anti-CTLA-4 treatment (at day 7). Mice were then monitored twice weekly for the development of subcutaneous tumors.

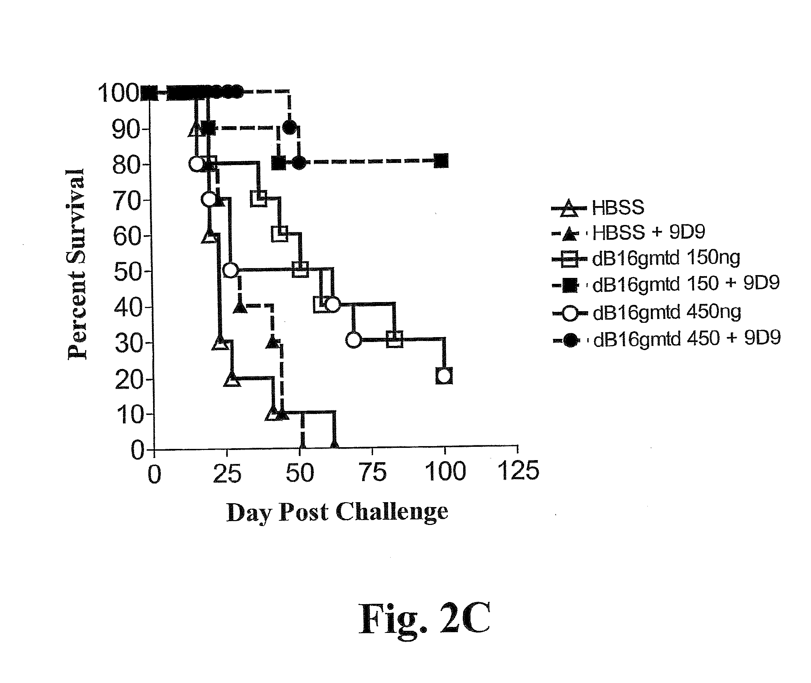

Figure 2C illustrates the results of a study where C57BI6 mice were challenged with 1 x 10⁵ B16F10 melanoma tumor cells. One day later, mice were vaccinated with 1 x10⁶ or 3 x 10⁶ irradiated GM-CSF-secreting B16F10 (dB16gmtd150 generating 150 ng of GM-CSF/1X10⁶ cells/24h and dB16gmtd450 generating 450 ng of GM-CSF/1X10⁶ cells/24h, respectively) melanoma tumor cells. One and three days after vaccination, mice were injected with 150 µg and 100 µg, respectively, of a murine anti-CTLA-4 monoclonal antibody (anti-CTLA-4 9D9). Mice received a second vaccination of 3 × 10⁶ irradiated cells two days after the last anti-CTLA-4 treatment and were based on survival time.

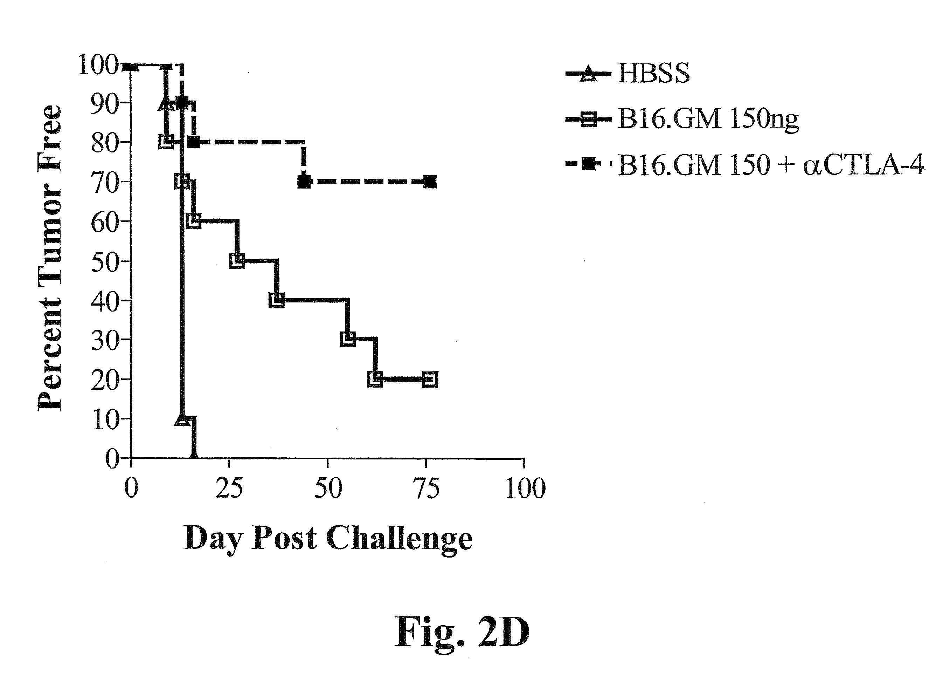

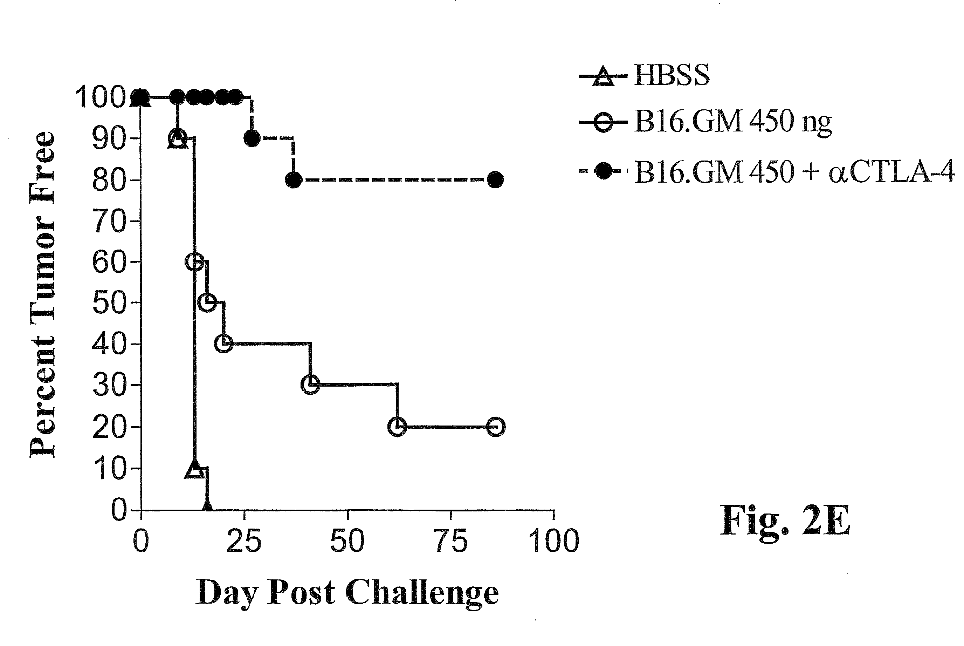

Figures 2D and 2E provide a graphic depiction of the results of a study such as that described in Fig 2A where the GM-CSF secretion level was 150 ng/10⁶ cells/24hours (low) or 450 ng/10⁶ cells/24hours (high), respectively.

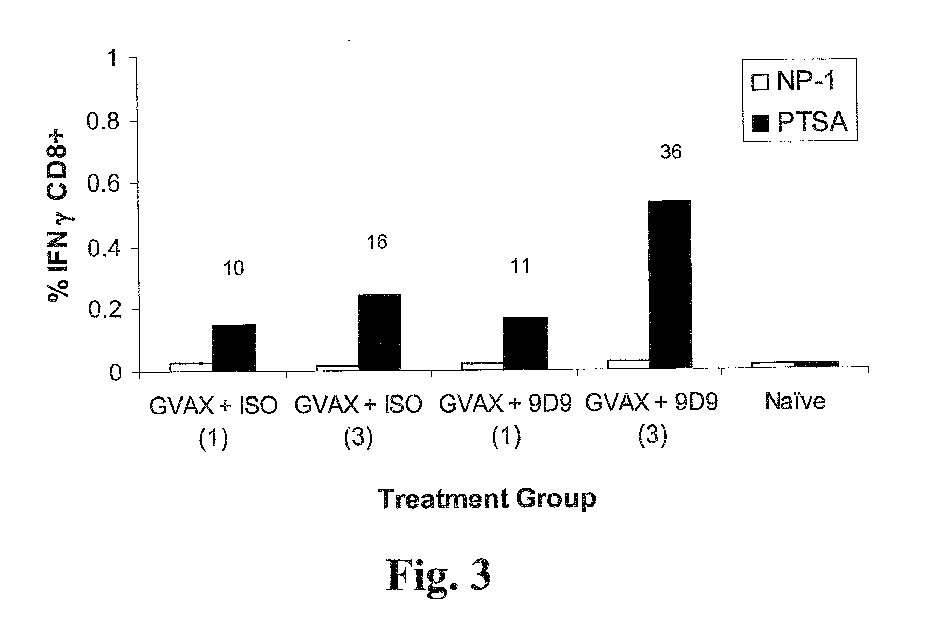

Figure 3 illustrates the results of a study where C57BI6 mice were vaccinated with GM-CSF-secreting murine prostate adenocarcinoma cells every two weeks for a total of five vaccinations. Mice were injected with a murine anti-CTLA-4 9D9 antibody or an isotype control antibody after the first vaccination only (1) or three times (3) at monthly intervals following vaccinations 1, 3, and 5. Two weeks after the last vaccination, spleen cells were analyzed by flow cytometry for prostate specific tumor antigen (PTSA)-specific intracellular IFN-gamma expression. The number above each bar represents the fold increase of PTSA-specific IFN-gamma⁺CD8⁺ T-cells from each treatment group compared to naïve mice. NP-1 is a non-specific peptide control.

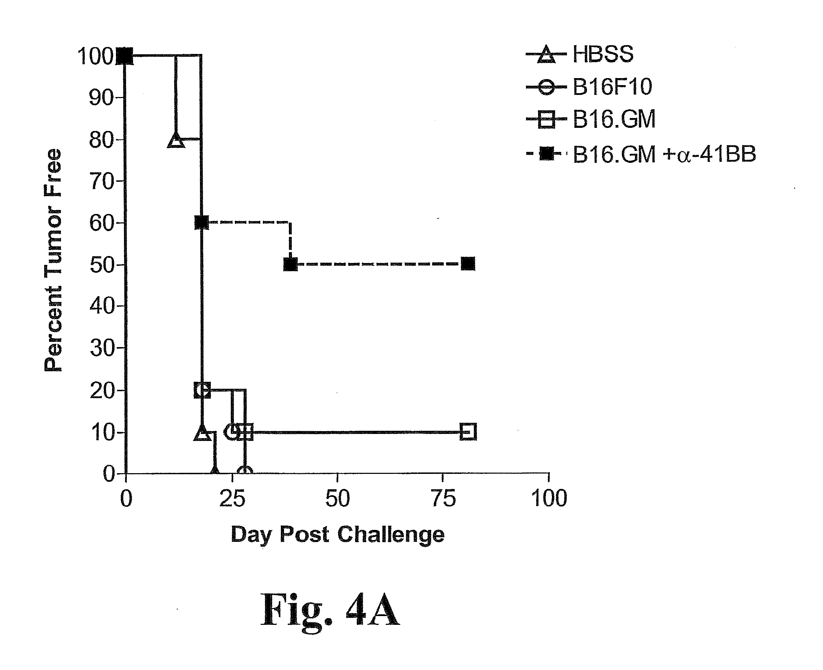

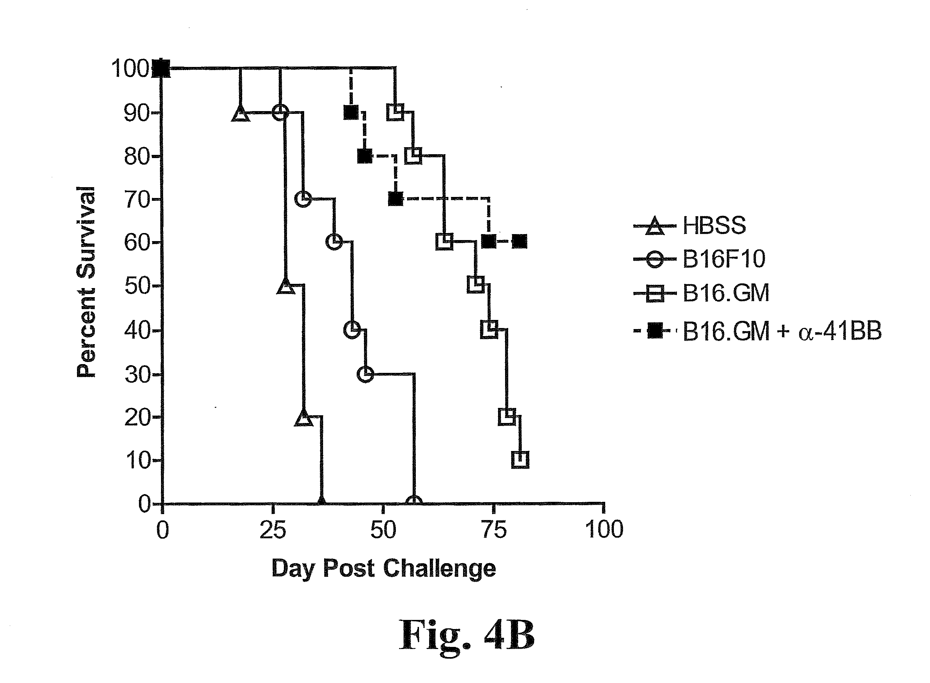

Figures 4A and 4B illustrate the results of a study in the B16F10 melanoma model wherein the effect of GVAX® was evaluated in combination with a 4-1BB monoclonal antibody. In the study C57BI6 mice were challenged with 1×10⁵ B16F10 tumor cells on Day 0, Three days later, mice were vaccinated with 3×10⁶ irradiated B16F10 or GM-CSF-secreting B16F10 cells. Three days after vaccination, mice were injected IP with 150ug 4-1BB monoclonal antibody. Mice were sacrificed on Day 21 and subjected to immunological monitoring. Fig. 4A illustrates the percentage of tumor-free mice and Fig. 4B illustrates percentage survival up to 80 days post challenge.

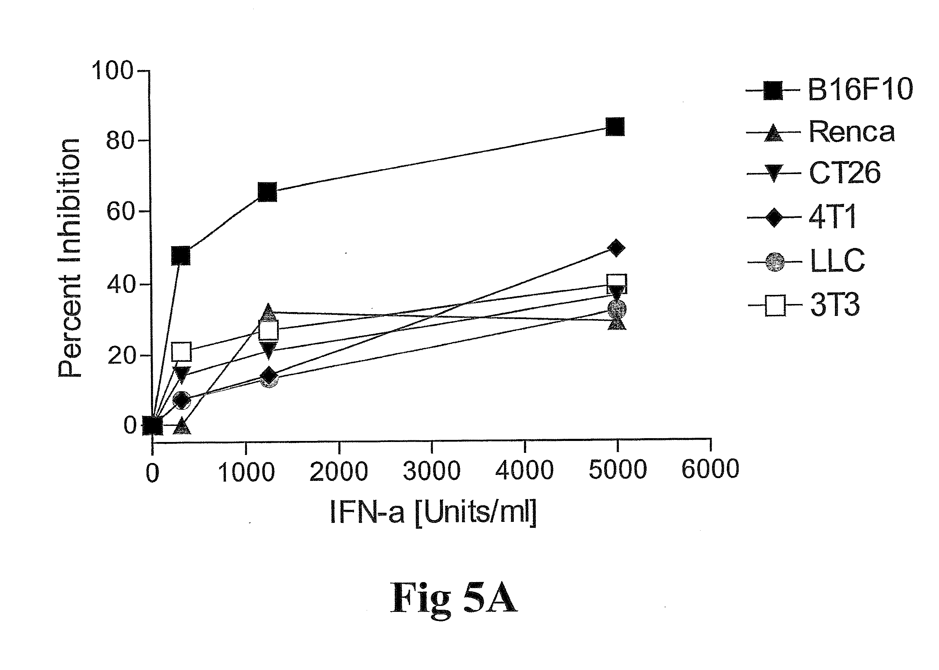

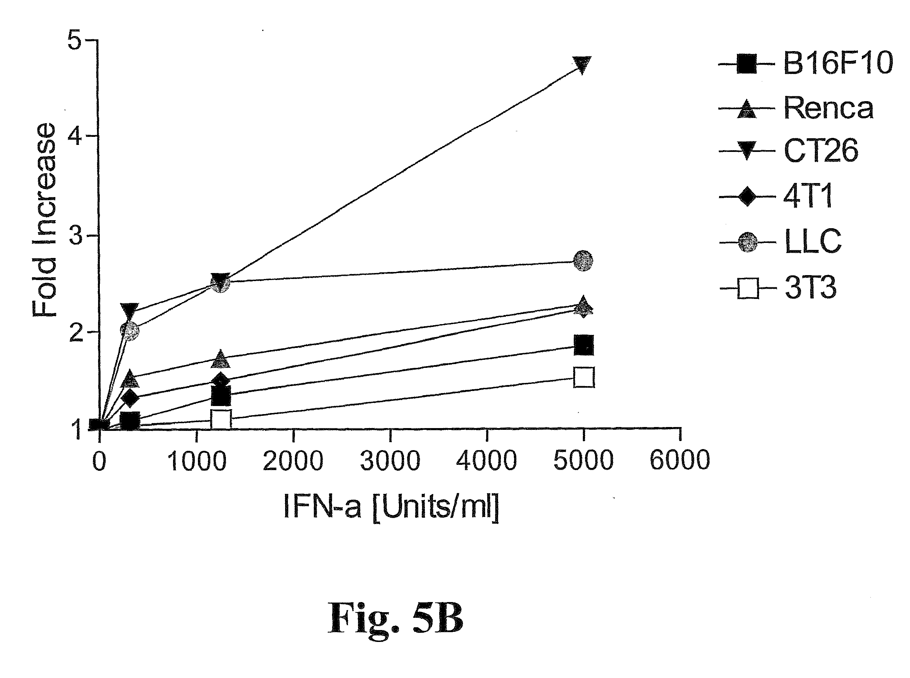

Figures 5A and B illustrate the results of in vitro studies with interferon-alpha.

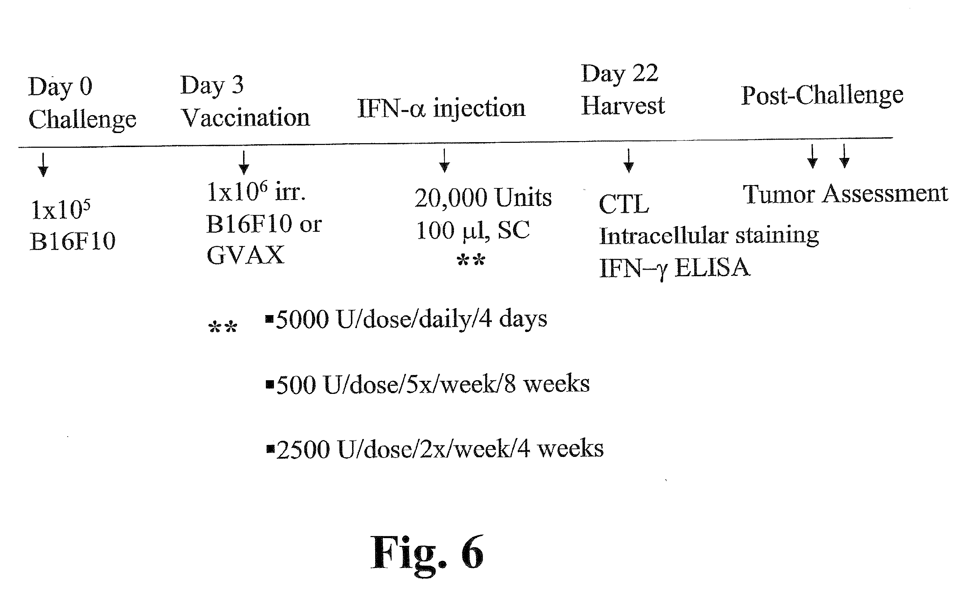

Figure 6 graphically represents the study design wherein the effect of the combination of GVAX® and interferon-alpha was evaluated in a murine B16F10 tumor model.

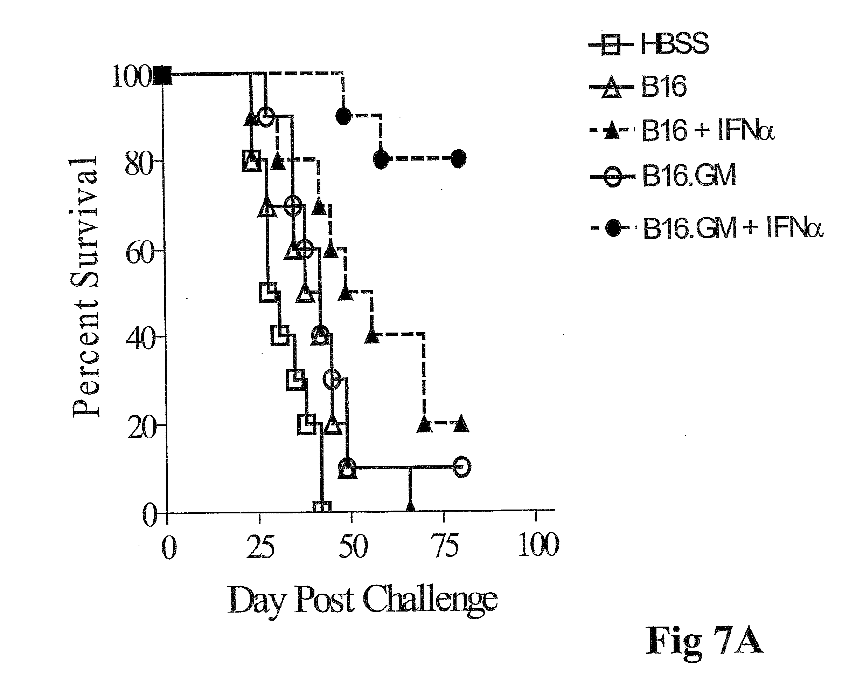

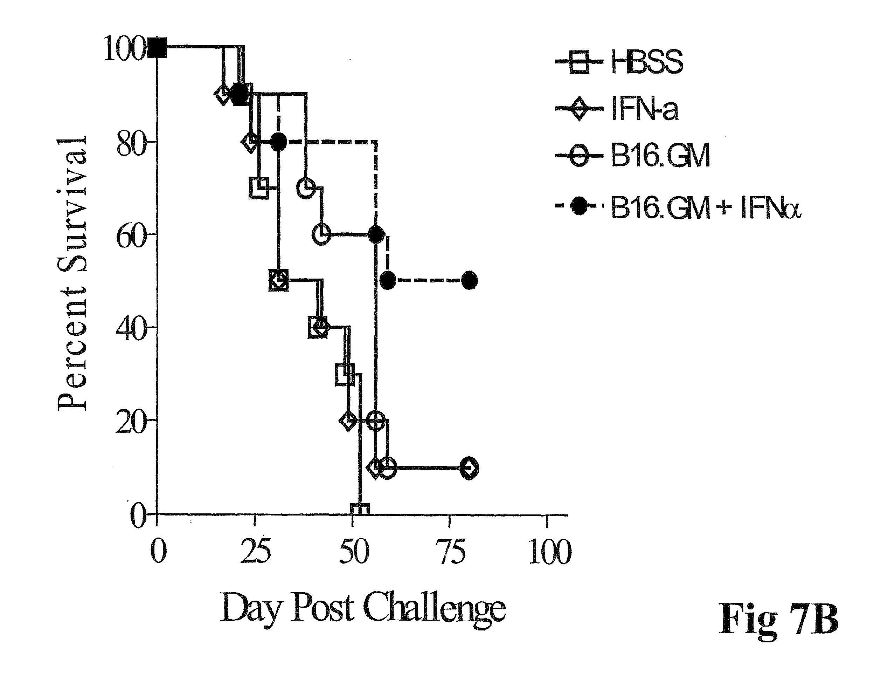

Figures 7A and B illustrate the results of in vivo studies with interferon-alpha where mice were treated with a combination of B16F10 or GM-C5F-secreting B16F10 (dB16gmtd) cells. The results shown in Fig 7A are derived from a study where on Day 0,10⁵ B16F10 melanoma cells were injected SC into the dorsal site of syngeneic C57/B6 mice as tumor challenge. On Day 3, irradiated B16.GM (10⁶) were injected SC into the ventral site as a cellular vaccine. On Day 7, IFN-alpha injections began. IFN-alpha injections were given as 2500 units twice/week for 4 weeks in 100ul, SC at the local tumor challenge site. The total IFN-alpha dose delivered was 20,000 Units. Tumor establishment was determined by palpation every 3-4 days. Mice with necrotic tumors or tumor size of 150mm² were sacrificed. Data shown is from 10 mice/study expressed as percentage of survival at the indicated time points. Survival efficacy of B16.GM was 40% and 816.GM + IFN-alpha was 40% above B16.GM monotherapy with the significance of p=0.08.

The results shown in Fig 7B are derived from a study where on Day 0,10⁵ B16F10 melanoma cells were injected SC into the dorsal site of syngeneic C57/B6 mice as tumor challenge. On Day 3, irradiated B16.GM (10⁶) were injected SC into the ventral site as a cellular vaccine. On Day 7, IFN-alpha injections began. IFN-alpha injections were given 2500 units twice/week for 4 weeks at 100ul, SC at the local vaccine site. The total IFN-alpha dose delivered was 20,000 Units. Tumor establishment was determined by palpation every 3-4 days. Mice with necrotic tumors or tumor size of 150mm² were sacrificed. Data shown is from 10 mice/study expressed as percentage of survival at the indicated time points. Survival efficacy of B16.GM was 10% and B16.GM + IFN-alpha was 40% above B16.GM monotherapy with the significance of (p=0.08).

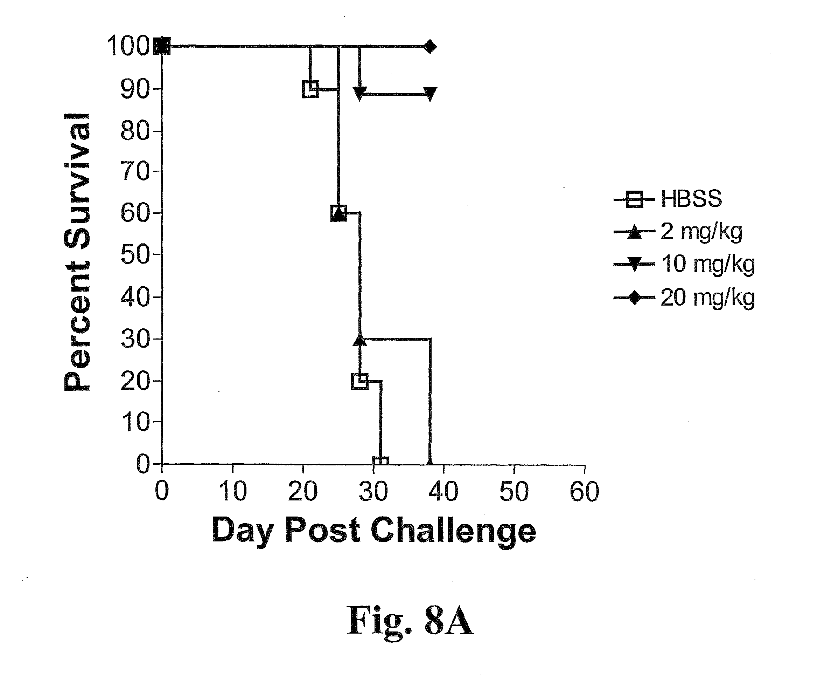

Figure 8A illustrates the results of a study in the B16F10 melanoma model wherein the effect of 3 IV injections of docetaxel (total dose 18 mg/kg) administered on days 5, 9, and 13 was evaluated with respect to the percentage of mice that survived.

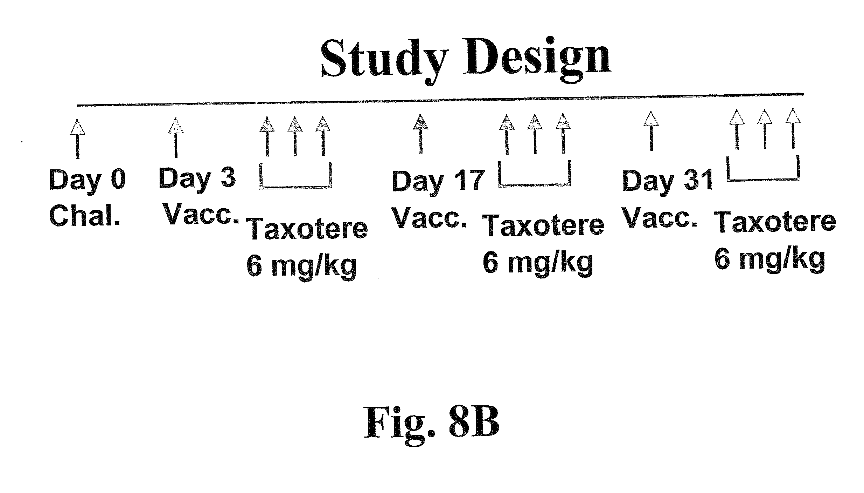

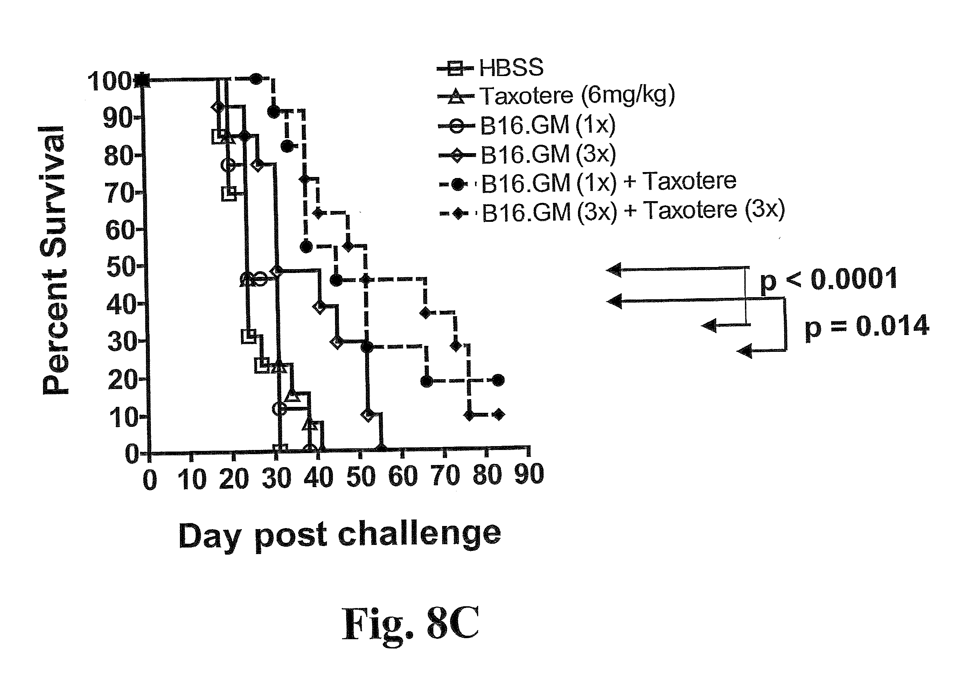

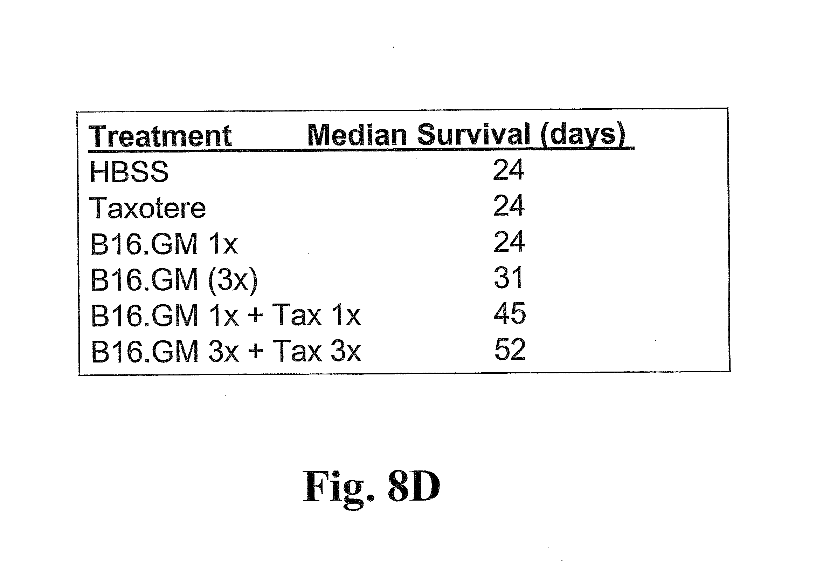

Figure 8B illustrates the study carried out to generate the data shown in Figs. 8C and D. The study was performed in the B16F10 melanoma model and compared the efficacy of one cycle of a CM-CSF secreting tumor cell vaccine administration combined with one cycle of Taxotere administration to three cycles of vaccine plus three cycles of taxotere.

Figures 8C and 8D illustrate the results of a study in the B16F10 melanoma model (carried out as described in Fig. 8B) wherein the effect of a cytokine-expressing cellular vaccine was evaluated in combination with docetaxel (total dose 18 mg/kg) administered on days 5, 9, and 13. The percentage of mice that survived are shown in Fig 8C and the median survival time is shown in Fig 8D, respectively. Administration of docetaxel given between bi-weekly vaccinations (three vaccinations total; three cycles) increased the median survival of B16 tumor-bearing mice from 31 days to 52 days when compared to three B16-GM vaccinations alone. The results of a study where one cycle of vaccine + docetaxel was administered increased the median survival time from 24 days (vaccine only) to 45 days (one cycle of vaccine + docetaxel).

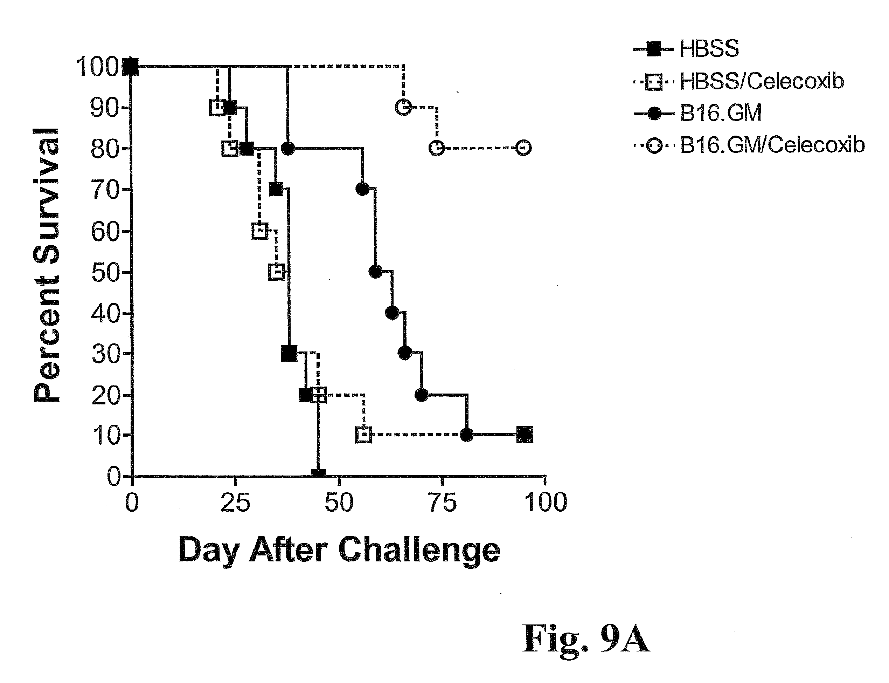

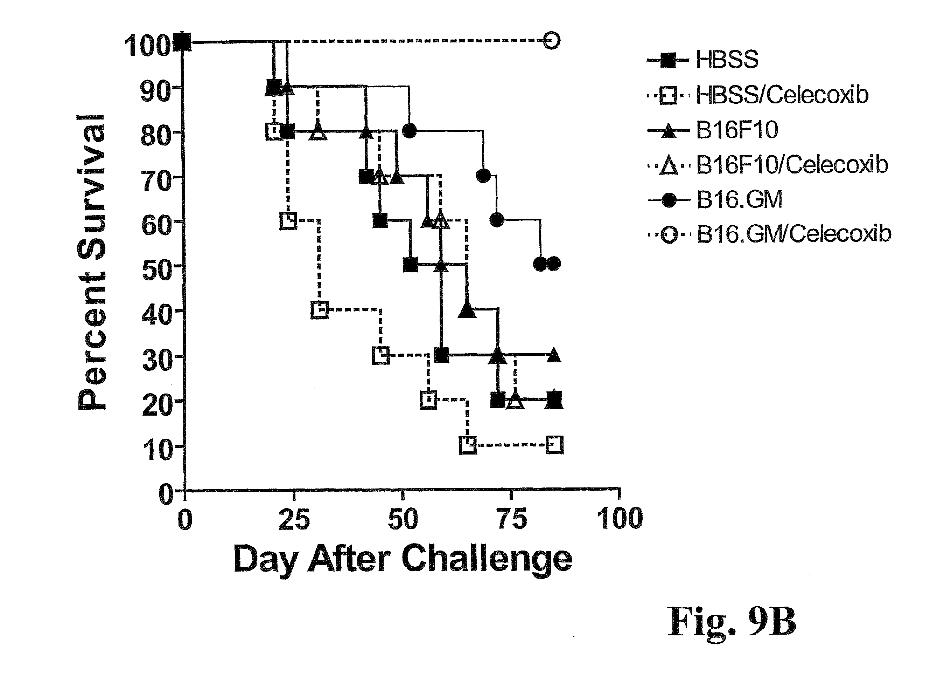

Figures 9A and 9B illustrate the results of a study in the B16F10 melanoma model wherein the effect of a cytokine-expressing cellular vaccine was evaluated in combination with Celebrex (Celecoxib; 50mg/kg/day in the feed starting on Day -7). Mice were challenged on Day 0 with 1×10⁵ live B16F10 cells at a dorsal location and were vaccinated on Day 3 with 1×10⁶ irradiated dB16gmtd cells at a ventral location. The results showed that Celebrex given 10 days before the GM-CSF secreting tumor cell vaccine increased the potency of the vaccine significantly resulting in long-term survival.

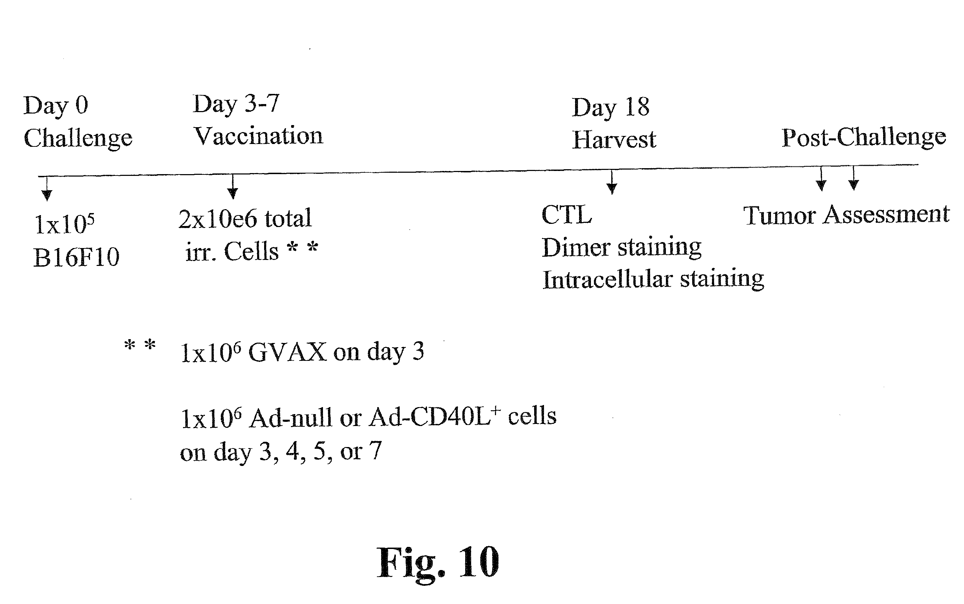

Figure 10 graphically represents the study design wherein the effect of the combination of GVAX® and CD40L was evaluated in a murine B16F10 model.

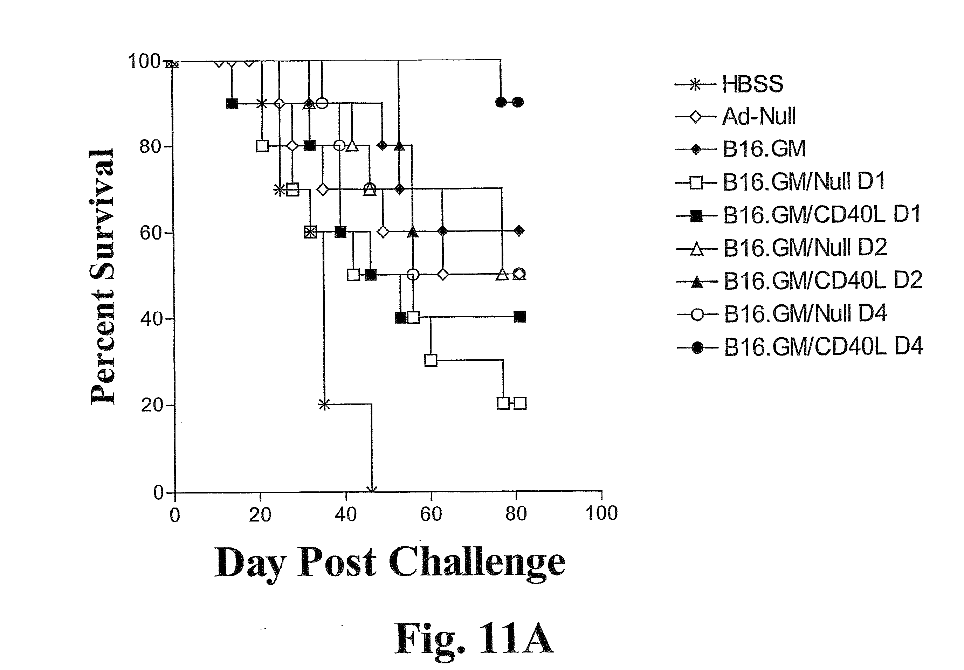

Figure 11A illustrates the results of treatment with the combination of B16F10 and adenovirus-expressed CD40 ligand in the B16F10 model. On day 0, mice were challenged with 1 x 10⁵ live B16F10 mouse melanoma cells, and on day 3 after challenge, mice were vaccinated ventrally with 1 x 10⁶ B16-CM, followed by administration of further irradiated vaccine cells on day 3, day 4, day 5, and day 7, as shown in Table 4. Mice were monitored for tumor burden every 3-4 days for 3 months (n=10) and sacrificed on day 18, with spleens and blood collected for immunological assays.

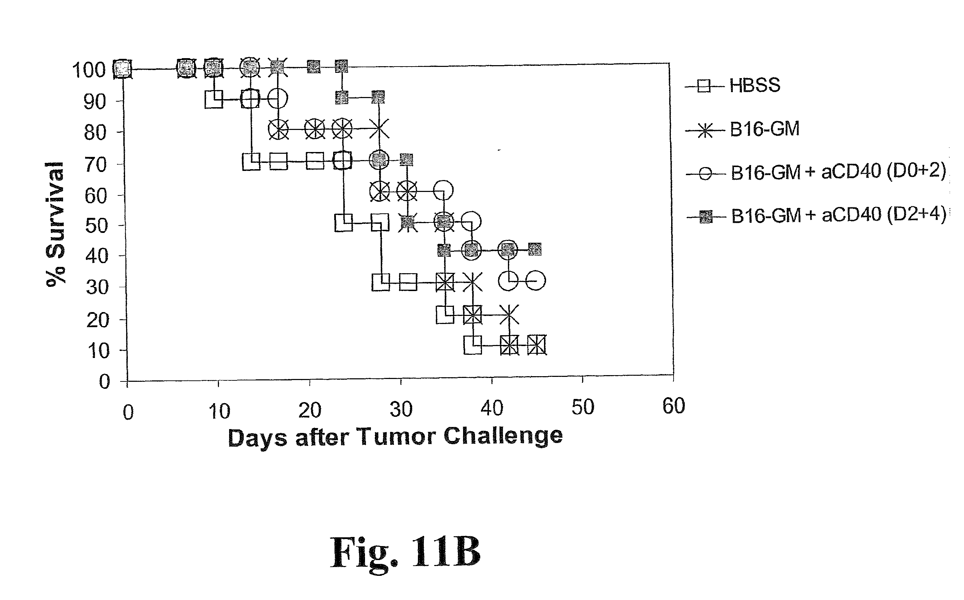

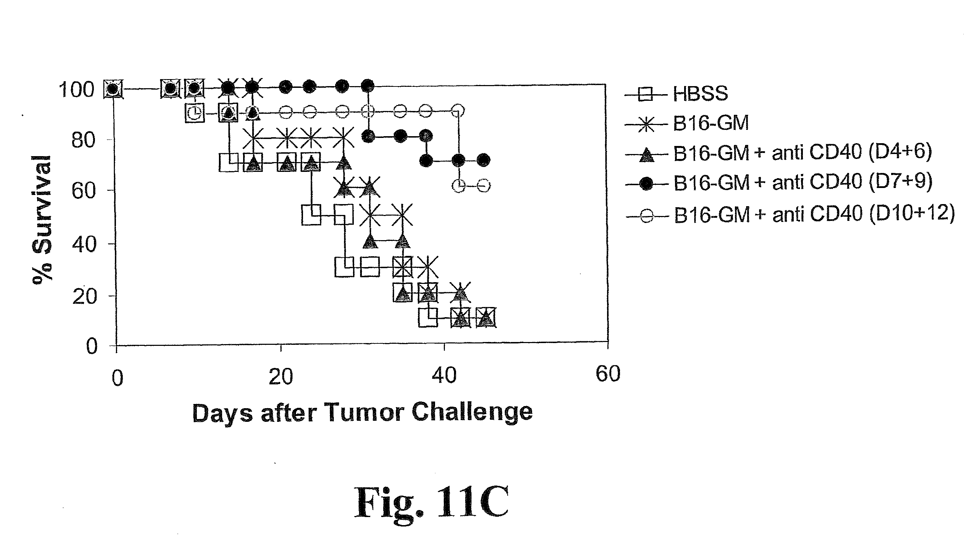

Figures 11B and C illustrate the results of treatment with the combination of B16F10 and an anti-CD40 antibody in the B16F10 model. C57BI6 mice were challenged dorsally with 1 x 10⁵ live B16F10 mouse melanoma cells. On day 3 after challenge mice were vaccinated ventrally with 1 x 10⁶ B16-CM. 100ug of anti CD40 antibody was administered per injection on D0 and 2 or D2 and 4 (Fig. 11B) or D4 and 6; D7 and 9 or D10 and 12 (Fig. 11C). Mice were monitored for tumor burden every 3-4 days for 3 months (n=10) and sacrificed on day 18, with spleens and blood collected for immunological assays.

### Detailed Description

The present invention represents improved cellular vaccines for the treatment of cancer in that the compositions and methods described herein comprise at least two components that act in concert to effect an improved therapeutic outcome for the patient under treatment.

### Definitions

The terms "regulating the immune response" or "modulating the immune response" as used herein refers to any alteration in a cell of the immune system or any alteration in the activity of a cell involved in the immune response. Such regulation or modulation includes an increase or decrease in the number of various cell types, an increase or decrease in the activity of these cells, or any other changes which can occur within the immune system. Cells involved in the immune response include, but are not limited to, T lymphocytes, B lymphocytes, natural killer (NK) cells, macrophages, eosinophils, mast cells, dendritic cells and neutrophils. In some cases, "regulating" or "modulating" the immune response means the immune response is stimulated or enhanced and in other cases "regulating" or "modulating" the immune response means suppression of the immune system. Stimulation of the immune system may include memory responses and/or future protection against subsequent antigen challenge.

The terms "cancer therapeutic agent", "additional cancer therapeutic agent or treatment" and the like as used herein refer to any molecule or treatment that stimulates an anti-cancer response when used in combination with a cytokine-expressing cellular vaccine such as GVAX®. In one aspect, the additional cancer therapeutic agent is expressed by a recombinant tumor cell and may be an immunomodulatory molecule, i.e. a second cytokine. In another aspect, the additional cancer therapeutic agent is administered in the form of a protein or other chemical entity, e.g., an antibody or standard chemotherapeutic agent such as taxotere, provided in a pharmaceutically acceptable excipient. In yet another aspect, the cancer therapeutic agent is a standard treatment traditionally used in the treatment of cancer, e.g., radiation. In a further aspect, the additional cancer therapeutic agent is an agent or treatment, which is typically not considered in the treatment of cancer, but which when administered to a patient in combination with a cytokine-expressing cellular vaccine results in an improved therapeutic outcome for the patient under treatment.

The term "cytokine" or "cytokines" as used herein refers to the general class of biological molecules which effect/affect cells of the immune system. The definition is meant to include, but is not limited to, those biological molecules that act locally or may circulate in the blood, and which, when used in the compositions or methods of the present invention serve to regulate or modulate an individual's immune response to cancer. Exemplary cytokines for use in practicing the invention include but are not limited to IFN-alpha, IFN-beta, and IFN-gamma, interleukins (e.g., IL-1 to IL-29, in particular, IL-2, IL-7, IL-12, IL-15 and IL-18), tumor necrosis factors (e.g., TNF-alpha and TNF-beta), erythropoietin (EPO), MIP3a, ICAM, macrophage colony stimulating factor (M-CSF), granulocyte colony stimulating factor (G-CSF) and granulocyte-macrophage colony stimulating factor (GM-CSF).

The term "cytokine-expressing cellular vaccine" as used herein refers to a composition comprising a population of cells that has been genetically modified to express a cytokine, e.g., GM-CSF, and that is administered to a patient as part of a cancer treatment regimen. The cells of such a "cytokine-expressing cellular vaccine" comprise a cytokine-encoding DNA sequence operably linked to expression and control elements such that the cytokine is expressed by the cells. The cells of the "cytokine-expressing cellular vaccine" are typically tumor cells and may be autologous or allogeneic to the patient undergoing treatment and or may be "bystander cells" that are mixed with tumor cells taken from the patient. A GM-CSF-expressing "cytokine-expressing cellular vaccine" may be referred to herein as "GVAX®".

The term "operably linked" as used herein relative to a recombinant DNA construct or vector means nucleotide components of the recombinant DNA construct or vector are directly linked to one another for operative control of a selected coding sequence, Generally, "operably linked" DNA sequences are contiguous, and, in the case of a secretory leader, contiguous and in reading frame, however, some sequences, e.g., enhancers do not have to be contiguous.

As used herein, the term "gene" or "coding sequence" means the nucleic acid sequence which is transcribed (DNA) and translated (mRNA) into a polypeptide in vitro or in vivo when operably linked to appropriate regulatory sequences. A "gene" typically comprises the coding sequence plus any non-coding sequences associated with the gene (e.g., regulatory sequences) and hence may or may not include regions preceding and following the coding region, e. g. 5' untranslated (5'UTR) or "leader" sequences and 3' UTR or "trailer" sequences, as well as intervening sequences (introns) between individual coding segments (exons). In contrast, a "coding sequence" does not include non-coding DNA.

The terms "gene-modified" and "genetically-modified" are used herein with reference to a cell or population of cells wherein a nucleic acid sequence has been introduced into the cell or population of cells. The nucleic acid sequence may be heterologous to the cell(s), or it may be an additional copy or improved version of a nucleic acid sequence already present in the cell(s), The cell(s) may be genetically-modified by physical or chemical methods or by the use of recombinant viruses. Chemical and physical methods such as calcium phosphate, electroporation and pressure mediated transfer of genetic material into cells are often used. Several recombinant viral vectors which find utility in effective delivery of genes into mammalian cells include, for example, retroviral vectors, adenovirus vectors, adenovirus-associated vectors (AAV), herpes virus vectors, pox virus vectors. In addition, non-viral means of introduction, for example, naked DNA delivered via liposomes, receptor-mediated delivery, calcium phosphate transfection, electroporation, particle bombardment (gene gun), or pressure-mediated delivery may also be employed to introduce a nucleic acid sequence into a cell or population of cells to render them "gene-modified" or "genetically-modified.

As used herein, the terms "tumor" and "cancer" refer to a cell that exhibits a loss of growth control and forms unusually large clones of cells. Tumor or cancer cells generally have lost contact inhibition and may be invasive and/or have the ability to metastasize.

The term "antigen from a tumor cell" and "tumor antigen" and "tumor cell antigen" may be used interchangeably herein and refer to any protein, carbohydrate or other component derived from or expressed by a tumor cell which is capable of eliciting an immune response. The definition is meant to include, but is not limited to, whole tumor cells that express all of the tumor-associated antigens, tumor cell fragments, plasma membranes taken from a tumor cell, proteins purified from the cell surface or membrane of a tumor cell, or unique carbohydrate moieties associated with the cell surface of a tumor cell. The definition also includes those antigens from the surface of the cell which require special treatment of the cells to access.

The term "systemic immune response" as used herein means an immune response which is not localized, but affects the individual as a whole.

The term "gene therapy" as used herein means the treatment or prevention of cancer by means of ex vivo or in vivo delivery, through viral or non-viral vectors, of compositions containing a recombinant genetic material.

The term "ex vivo" delivery as used herein means the introduction, outside of the body of a human, of compositions containing a genetic material into a cell, tissue, organoid, organ, or the like, followed by the administration of cell, tissue, organoid, organ, or the like which contains such introduced compositions into the body of the same (autologous) or a different (allogeneic) human, without limitation as to the formulation, site or route of administration.

The terms "inactivated cells", "non-dividing cells" and "non-replicating cells" may be used interchangeably herein and refer to cells that have been treated rendering them proliferation incompetent, e.g., by irradiation. Such treatment results in cells that are unable to undergo mitosis, but retain the capability to express proteins such as cytokines or other cancer therapeutic agents. Typically a minimum dose of about 3500 rads is sufficient, although doses up to about 30,000 rads are acceptable. Effective doses include, but are not limited to 5000 to 10000 rads. Numerous methods of inactivating cells, such as treatment with Mitomycin C, are known in the art. Any method of inactivation which renders cells incapable of cell division, but allows the cells to retain the ability to express proteins is included within the scope of the present invention.

As used herein "treatment" of an individual or a cell is any type of intervention used in an attempt to alter the natural course of the individual or cell. Treatment includes, but is not limited to, administration of e.g., a cytokine-expressing cellular vaccine and at least one additional cancer therapeutic agent or treatment, and may be performed either prophylactically or subsequent to diagnosis as part of a primary or follow-up therapeutic regimen.

The term "administering" as used herein refers to the physical introduction of a composition comprising a cytokine-expressing cellular vaccine and at least one additional cancer therapeutic agent or treatment to a patient with cancer. Any and all methods of introduction are contemplated according to the invention, the method is not dependent on any particular means of introduction and is not to be so construed. Means of introduction are well-known to those skilled in the art, examples of which are provided herein.

The term "co-administering" as used herein means a process whereby the combination of a cytokine-expressing cellular vaccine and at least one additional cancer therapeutic agent or treatment is administered to the same patient. The cytokine-expressing cellular vaccine and additional cancer therapeutic may be administered simultaneously, at essentially the same time, or sequentially. If administration takes place sequentially, the cytokine-expressing cellular vaccine may be administered before or after a given additional cancer therapeutic agent or treatment. The cytokine-expressing cellular vaccine and additional cancer therapeutic agent or treatment need not be administered by means of the same vehicle, the cellular vaccine and the additional agent or treatment may be administered one or more times and the number of administrations of each component of the combination may be the same or different. In addition, the cytokine-expressing cellular vaccine and additional cancer therapeutic agent or treatment need not be administered at the same site.

The term "therapeutically effective amount" or "therapeutically effective combination" as used herein refers to an amount or dose of a cytokine-expressing cellular vaccine together with the amount or dose of an additional agent or treatment that is sufficient to modulate, either by stimulation or suppression, the systemic immune response of an individual. The amount of cytokine-expressing cellular vaccine in a given therapeutically effective combination may be different for different individuals, different tumor types and will be dependent upon the one or more additional agents or treatments included in the combination. The "therapeutically effective amount" is determined using procedures routinely employed by those of skill in the art such that an "improved therapeutic outcome" results.

As used herein, the terms "improved therapeutic outcome" and "enhanced therapeutic efficacy", relative to cancer refers to a slowing or diminution of the growth of cancer cells or a solid tumor, or a reduction in the total number of cancer cells or total tumor burden. An "improved therapeutic outcome" or "enhanced therapeutic efficacy" therefore means there is an improvement in the condition of the patient according to any clinically acceptable criteria, including an increase in time to tumor progression, an increase in life expectancy, or an improvement in quality of life.

The term "reversal of an established tumor" as used herein means the suppression, regression, partial or complete disappearance of a pre-existing tumor. The definition is meant to include any diminution in the size, growth rate, appearance or cellular compositions of a preexisting tumor.

The terms "individual", "subject" as referred to herein is a vertebrate, preferably a mammal, and typically refers to a human.

### General Techniques

The practice of the present invention will employ, unless otherwise indicated, conventional techniques of molecular biology, microbiology, cell biology, biochemistry and immunology, which are within the knowledge of those of skill of the art. Such techniques are explained fully in the literature, such as, "Molecular Cloning: A Laboratory Manual", second edition (Sambrook et al., 1989); "Current Protocols in Molecular Biology" (F. M. Ausubel et al., eds., 1987); "Animal Cell Culture" (R. I. Freshney, ed., 1987), each of which is hereby expressly incorporated herein by reference.

### Cancer Targets

The methods and compositions of the invention provide an improved therapeutic approach to the treatment of cancer by co-administration of a cytokine-expressing cellular vaccine and at least one additional cancer therapeutic agent or treatment to a patient with cancer. "Cancer" as used herein includes cancer localized in tumors, as well as cancer not localized in tumors, such as, for instance, cancer cells that expand from a local tumor by invasion (i.e., metastasis). The invention finds utility in the treatment of any form of cancer, including, but not limited to, cancer of the bladder, breast, colon, kidney, liver, lung, ovary, cervix, pancreas, rectum, prostate, stomach, epidermis; a hematopoietic tumor of lymphoid or myeloid lineage; a tumor of mesenchymal origin such as a fibrosarcoma or rhabdomyosarcoma; other tumor types such as melanoma, teratocarcinoma, neuroblastoma, glioma, adenocarcinoma and non-small lung cell carcinoma.

### Introduction Of Cytokine And Cancer Therapeutic Agent-Encoding, Nucleic Acid Sequences Into Cells

In one aspect of the invention, a nucleic acid sequence (i.e., a recombinant DNA construct or vector) encoding a cytokine operably linked to a first promoter, alone or in combination with a nucleic acid sequence encoding a cancer therapeutic agent operably linked to a second promoter is introduced into a cell or population of cells. Any and all methods of introduction into a cell or population of cells, typically tumor cells, are contemplated according to the invention, the method is not dependent on any particular means of introduction and is not to be so construed. The cytokine-encoding nucleic acid sequence may be introduced into the same or a different population of cells as the cancer therapeutic agent-encoding nucleic acid sequence.

The "vector" may be a DNA molecule such as a plasmid, virus or other vehicle, which contains one or more heterologous or recombinant DNA sequences, e.g., a nucleic acid sequence encoding a cytokine or additional cancer therapeutic agent under the control of a functional promoter and in some cases further including an enhancer that is capable of functioning as a vector, as understood by those of ordinary skill in the art. An appropriate viral vector includes, but is not limited to, a retrovirus, a lentivirus, an adenovirus (AV), an adeno-associated virus (AAV), a simian virus 40 (SV-40), a bovine papilloma virus, an Epstein-Barr virus, a herpes virus, a vaccinia virus, a Moloney murine leukemia virus, a Harvey murine sarcoma virus, a murine mammary tumor virus, and a Rous sarcoma virus. Non-viral vectors are also included within the scope of the invention.

Any suitable vector can be employed that is appropriate for introduction of nucleic acids into eukaryotic tumor cells, or more particularly animal tumor cells, such as mammalian, e.g., human, tumor cells. Preferably the vector is compatible with the tumor cell, e.g., is capable of imparting expression of the coding sequence for a cytokine or cancer therapeutic agent, and is stably maintained or relatively stably maintained in the tumor cell. Desirably the vector comprises an origin of replication and the vector may or may not also comprise a "marker" or "selectable marker" function by which the vector can be identified and selected. While any selectable marker can be used, selectable markers for use in such expression vectors are generally known in the art and the choice of the proper selectable marker will depend on the host cell. Examples of selectable marker genes which encode proteins that confer resistance to antibiotics or other toxins include ampicillin, methotrexate, tetracycline, neomycin (Southern and Berg, J., 1982), mycophenolic acid (Mulligan and Berg, 1980), puromycin, zeomycin, hygromycin (Sugden et al., 1985) or G428.

In practicing the methods of the present invention, a vector comprising a nucleic acid sequence encoding a cytokine or additional cancer therapeutic agent may be transferred to a cell in vitro, preferably a tumor cell, using any of a number of methods which include but are not limited to electroporation, membrane fusion with liposomes, Lipofectamine treatment, high velocity bombardment with DNA-coated microprojectiles, incubation with calcium phosphate-DNA precipitate, DEAE-dextran mediated transfection, infection with modified viral nucleic acids, direct microinjection into single cells, etc. Procedures for the cloning and expression of modified forms of a native protein using recombinant DNA technology are generally known in the art, as described in Ausubel, et al., 1992 and Sambrook, et al., 1989, expressly incorporated by reference, herein.

Reference to a vector or other DNA sequences as "recombinant" merely acknowledges the operable linkage of DNA sequences which are not typically operably linked as isolated from or found in nature. A "promoter" is a DNA sequence that directs the binding of RNA polymerase and thereby promotes RNA synthesis. Enhancers" are cis-acting elements that stimulate or inhibit transcription of adjacent genes. An enhancer that inhibits transcription also is termed a "silencer". Enhancers can function (i.e. be operably linked to a coding sequence) in either orientation, over distances of up to several kilobase pairs (kb) from the coding sequence and from a position downstream of a transcribed region. Regulatory (expression/control) sequences are operatively linked to a nucleic acid coding sequence when the expression/control sequences regulate the transcription and, as appropriate, translation of the nucleic acid sequence. Thus expression/control sequences can include promoters, enhancers, transcription terminators, a start codon (i. e., ATG) in front of the coding sequined, splicing signal for introns and stop codons.

Recombinant vectors for the production of cellular vaccines of the invention provide all the proper transcription, translation and processing signals (e.g., splicing and polyadenylation signals) such that the coding sequence for the cytokine or cancer therapeutic agent is appropriately transcribed and translated in the tumor cells into which the vector is introduced. The manipulation of such signals to ensure appropriate expression in host cells is within the skill of the ordinary skilled artisan. The coding sequence for the cytokine and cancer therapeutic agent may be under control of (i.e., operably linked to) it's own native promoter, or a non-native (i.e. heterologous) promoter, including a constitutive promoter, e.g., the cytomegalovirus (CMV) immediate early promoter/enhancer, the Rous sarcoma virus long terminal repeat (RSV-LTR) or the SV-40 promoter.

Alternately, a tissue-specific promoter (a promoter that is preferentially activated in a particular type of tissue and results in expression of a gene product in that tissue) can be used in the vector. Such promoters include but are not limited to a liver specific promoter (Ill CR, et al., Blood Coagul Fibrinolysis 8 Suppl 2:523-30, 1997) and the EF-1 alpha promoter (Kim DW et al. Gene. 91(2):217-23, 1990, Guo ZS et al. Gene Ther. 3(9):802-10, 1996; US Pat. Nos. 5,266,491 and 5,225,348, each of which expressly incorporated by reference herein). Inducible promoters also find utility in practicing the methods described herein, such as a promoter containing the tet responsive element (TRE) in the tet-on or tet-off system as described (ClonTech and BASF), the metallothienein promoter which can be upregulated by addition of certain metal salts and rapamycin inducible promoters (Rivera et al., 1996, Nature Med, 2(9):1028-1032; Ye et al., 2000, Science 283: 88-91; Sawyer TK et al., 2002, Mini Rev Med Chem. 2(5):475-88). Large numbers of suitable tissue-specific or regulatable vectors and promoters for use in practicing the current invention are known to those of skill in the art and many are commercially available.

Exemplary vector systems for use in practicing the invention include the retroviral MFG vector, described in U.S. Pat. No. 5,637,483, expressly incorporated by reference herein. Other useful retroviral vectors include pLJ, pEm and [alpha]SGC, described in U.S. Pat. No. 5,637,483 (in particular Example 12), U.S. Pat. Nos. 6,506,604, 5,955,331 and USSN 09/612808, each of which is expressly incorporated by reference herein.

Further exemplary vector systems for use in practicing the invention include second, third and fourth generation lentiviral vectors, US Pat. Nos. 6,428,953, 5,665,577 and 5,981,276 and WO 00/72686, each of which is expressly incorporated by reference herein.

Additional exemplary vector systems for use in practicing the present invention include adenoviral vectors, described for example in U.S. Pat. No. 5,872,005 and WO 00/72686, each of which is expressly incorporated by reference herein.

Yet another vector system that is preferred in practicing the methods described herein is a recombinant adeno-associated vector (rAAV) system, described for example in WO98/46728, WO 00/72686, Samulski et al., Virol. 63:3822-3828 (1989) and US Pat. Nos. 5436146, 5753500, 6037177, 6040183 and 6093570, each of which is expressly incorporated by reference herein.

### Cytokines

Cytokines and combinations of cytokines have been shown to play an important role in the stimulation of the immune system. The term "cytokine" is understood by those of skill in the art, as referring to any immunopotentiating protein (including a modified protein such as a glycoprotein) that enhances or modifies the immune response to a tumor present in the host. The cytokine typically enhances or modifies the immune response by activating or enhancing the activity of cells of the immune system and is not itself immunogenic to the host.

It follows from the results presented herein that a variety of cytokines will find use in the present invention. Exemplary cytokines for use in practicing the invention include but are not limited to IFN-alpha, IFN-beta, and IFN-gamma, interleukins (e.g., IL-1 to IL-29, in particular, IL-2, IL-7, IL-12, IL-15 and IL-18), tumor necrosis factors (e.g., TNF-alpha and TNF-beta), erythropoietin (EPO), MIP3a, macrophage colony stimulating factor (M-CSF), granulocyte colony stimulating factor (G-CSF) and granulocyte-macrophage colony stimulating factor (CM-CSF). The cytokine may be from any source, however, optimally the cytokine is of murine or human origin (a native human or murine cytokine) or is a sequence variant of such a cytokine, so long as the cytokine has a sequence with substantial homology to the human form of the cytokine and exhibits a similar activity on the immune system. It follow that cytokines with substantial homology to the human forms of IFN-alpha, IFN-beta, and IFN-gamma, IL-1 to IL-29, TNF-alpha, TNF-beta, EPO, MIP3a, ICAM, M-CSF, G-CSF and GM-CSF are useful in practicing the invention, so long as the homologous form exhibits the same or a similar effect on the immune system. Proteins that are substantially similar to any particular cytokine, but have relatively minor changes in protein sequence find use in the present invention. It is well known that small alterations in protein sequence may not disturb the functional activity of a protein molecule, and thus proteins can be made that function as cytokines in the present invention but differ slightly from current known or native sequences.

### Variant Sequences

Homologues and variants of native human or murine cytokines and additional cancer therapeutic agent are included within the scope of the invention. As used herein, the term "sequence identity" means nucleic acid or amino acid sequence identity between two or more aligned sequences and is typically expressed as a percentage ("%"). The term "% homology" is used interchangeably herein with the term "% identity" or "% sequence identity" and refers to the level of nucleic acid or amino acid sequence identity between two or more aligned sequences, when aligned using a sequence alignment program. For example, as used herein, 80% homology means the same thing as 80% sequence identity determined by a defined algorithm, and accordingly a homologue of a given sequence typically has greater than 80% sequence identity over a length of the given sequence. Preferred levels of sequence identity include, but are not limited to, 80, 85, 88, 89, 90, 91, 92, 93, 94, 95, 96, 97, 98 or 99% or more sequence identity to a native cytokine or cancer therapeutic agent amino acid or nucleic acid sequence, as described herein.

Exemplary computer programs that can be used to determine the degree of identity between two sequences include, but are not limited to, the suite of BLAST programs, e.g., BLASTN, BLASTX, TBLASTX, BLASTP and TBLASTN, all of which are publicly available on the Internet. See, also, Altschul, S. F. et al. Mol. Biol. 215:403-410,1990 and Altschul, S. F. et al. Nucleic Acids Res. 25:3389-3402, 1997, expressly incorporated by reference herein. Sequence searches are typically carried out using the BLASTN program when evaluating a given nucleic acid sequence relative to nucleic acid sequences in the GenBank DNA Sequences and other public databases. The BLASTX program is preferred for searching nucleic acid sequences that have been translated in all reading frames against amino acid sequences in the GenBank Protein Sequences and other public databases. In determining sequence identity, both BLASTN and BLASTX (i.e. version 2.2.5) are run using default parameters of an open gap penalty of 11.0, and an extended gap penalty of 1.0, and utilize the BLOSUM-62 matrix. [See, Altschul, et al., 1997, supra.] A preferred alignment of selected sequences in order to determine "% identity" between two or more sequences, is performed using for example, the CLUSTAL-W program in MacVector version 6.5, operated with default parameters, including an open gap penalty of 10.0, an extended gap penalty of 0.1, and a BLOSUM 30 similarity matrix.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about TM-5°C (5° below the Tm of the probe) "high stringency" at about 5-10° below the Tm; "intermediate stringency" at about 10-20° below the Tm of the probe; and "low stringency" at about 20-25° below the Tm. Functionally, maximum stringency conditions may be used to identify sequences having strict identity or near-strict identity with the hybridization probe, while high stringency conditions are used to identify sequences having about 80% or more sequence identity with the probe. An example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. Moderate and high stringency hybridization conditions are well known in the art. See, for example, Sambrook, et al,1989, Chapters 9 and 11, and in Ausubel, F. M., et al., 1993, (expressly incorporated by reference herein).

### One Or More Additional Agents Or Treatments

As detailed herein, the present invention is directed to a method of improving an individual's immune response to cancer (e.g., a target cancer antigen or antigens) by co-administering a cytokine-expressing cellular vaccine (e.g., GM-CSF; GVAX®) and at least one additional cancer therapeutic agent or treatment to a patient with cancer. Cancer therapeutic agents or treatments for use in practicing the invention include, but are not limited to, adhesion or accessory molecules, other biological response modifiers, chemotherapeutic agents, radiation treatment and combinations thereof.

### Cancer Therapeutic Agents

Embodiments of the present invention include the administration of the combination of a cytokine-expressing cellular vaccine and at least one additional cancer therapeutic agent. Cancer therapeutic agents for use in practicing the invention include those listed in Tables 1A and 1B. These include agents from each of the major classes of cancer therapeutic agents, including but not limited to: alkylating agents, alkaloids, antimetabolites, anti-tumor antibiotics, nitrosoureas, hormonal agonists/antagonists and analogs, immunomodulators, photosensitizers, enzymes and others. In some preferred embodiments, the cancer therapeutic agent is a cytokine or co-stimulatory molecule. In other preferred embodiments the cancer therapeutic agent is for example CpG (a CG dinucleotide that mimics bacterial DNA); a HSP (heat shock protein); a Cox-2 inhibitor; an anti-41BB mAb; IL-2, IL-18; an anti-CTLA-4 monoclonal antibody; an anti-CD40 or membrane/soluble CD40 ligand; interferon alpha (IFN-alpha); Iressa; anti-epidermal growth factor R (EGF-R); or anti-VEGF R. Preferred cancer therapeutic agents include, but are not limited to, 5-fluorouracil, cisplatin, doxorubicin, estramustine, etoposide, mitoxantrone, docetaxel (TAXOTERE™), paclitaxel (TAXOL™), and retro-gutless adeno, IL-15, chemo-taxotere and erastomustin (plus steroids).

**Table 1A: Cancer Therapeutic Agents**

| ALKALOIDS | ALKYLATING AGENTS | ANTIBIOTICS AND ANALOGS | ANTIMETABOLITE S | ENZYMES | IMMUNOMODULATOR S |
|---|---|---|---|---|---|
| Docetaxel (TAXOTERE™) | alkyl Sulfonates | Aclacinomycins | Folic Acid Analogs | L-Asparaginase | Interferon-α |
| Etoposide | Busulfan | Actinomycin F₁ | Denopterin | Pegasargase | Interferon-β |
| Irinotecan | Improsulfan | Anthramycin | Edatrexate | | Interferon-γ |
| Paclitaxel (TAXOL™) | Piposulfan | Azaserine | Methotrexate | | Interferon-alpha-2a |
| Teniposide | | Bleomycins | Piritrexim | | Interleukin-2 |
| Topotecan | Aziridines | Cactinamycin | Pteropterin | | Lentinan |
| Vinblastine | Benzodepa | Carubicin | Tomudex® | | Propagermanium |
| Vincristine | Carboquone | Carzinophilin | Trimetrexate | | PSK |
| Vendesine | Meturedepa | Chromomycins | | | Roquinimex |
| Vinorelbine | Uredepa | Dactinomycin | Purine Analogs | | Rituximab |
| | | Daunorubicin | Cladribine | | Sizofiran |
| | Ethylenimines and Methylmelamine s | 6-Diazo-5-oxo-L-norleucine | Fludarabine | | Trastuzumab |
| | Altretamine | Doxorubicin | 6-Mercaptopurine | | Ubenimex |
| | Triethylenemela mine | Epirubicin | Thiamiprine | | Cyclophosphamide/ cytoxan |
| | Triethylenephos phoramide | Idarubicin | Thioguanine | | |
| | Triethylenethiop hosphoramide | Menogaril | | | |
| | | Mitomycins | | | |
| | | Mitoxantrone | Pyrimidine Analogs | | |
| | Nitrogen Mustards | Mycophenolic Acid | Ancitabine | | |
| | Chlorambucil | Nogalamycin | 5-Azacytidine | | |
| | Chlomaphazine | Olivomycins | 6-Azauridine | | |
| | Cyclophosphamid e | Peplomycin | Carmofur | | |
| | Estramustine | Pirarubicin | Cytarabine | | |
| | Ifosfamide | Plicamycin, | Doxifluridine | | |
| | Mechlorethamin e | Porfiromycin | Emitefur | | |
| | Mechlorethamin e Oxide Hydrochloride | Puromycin | Enocitabine | | |
| | Melphalan | Streptonigrin | Floxuridine | | |
| | Novembichin | Streptozocin | Fluorouracil | | |
| | | Valrubicin | | | |
| | Perfosfamide | Tubercidin | Gemcitabine | | |
| | Phenesterine | Zinostatin | Tegafur | | |
| | Prednimustine | Zorubicin | | | |
| | Trofosfamide | | | | |
| | Uracil Mustard | | | | |
| | Carboplatin | | | | |
| | Cisplatin | | | | |
| | Miboplatin | | | | |
| | Oxaliplatin | | | | |
| | | | | | |
| | Others | | | | |
| | Dacarbazine | | | | |
| | Mannomustine | | | | |
| | Mitobronitol | | | | |
| | Mitolactol | | | | |
| | Thiotepa | | | | |
| | Pipobroman | | | | |
| | Temozolomide | | | | |

**Table 1B: CANCER THERAPEUTIC AGENTS**

| NITROSOUREAS | OTHERS | HORMONE ANTAGONISTS/AGONISTS & ANALOGS | PHOTOSENSITIZER |
|---|---|---|---|
| Carmustine | Aceglatone | Dexamethasone | Porfimer Sodium |
| Chlorozotocin | Amsacrine | Prednisone | |
| Fotemustine | Bisantrene | | |
| Lomustine | Defosfamide | Androgens | |
| Nimustine | Demecolcine | Calusterone | |
| Ranimustine | Diaziquone | Dromostanolone | |
| | Eflornithine | Epitiostanol | |
| | Elliptinium Acetate | Mepitiostane | |
| | Etoglucid | Testolactone | |
| | Fenretinide | | |
| | Finasteride | Antiadrenals | |
| | Gallium Nitrate | Aminoglutethimide | |
| | Hydroxyurea | Mitotane | |
| | Lonidamine | Trilostane | |
| | Miltefosine | | |
| | Mitoguazone | Antiandrogens | |
| | Mopidamol | Bicalutamide | |
| | Nitracrine | Flutamide | |
| | Pentostatin | Nilutamide | |
| | Phenamet | | |
| | Podophyllinic Acid 2-Ethylhydrazide | Antiestrogens | |
| | Procarbazine | Droloxifene | |
| | Razoxane | Tamoxifen | |
| | Sobuzoxane | Toremifene | |
| | Spirogermanium | Exemestane | |
| | Amsacrine | Aromatase Inhibitors | |
| | Tretinoin | Aminoglutethimide | |
| | Tenuazonic Acid | Anastrozole | |
| | Triaziquone | Fadrozole | |
| | 2,2',2"-Triclorotriethyla mine, | Formestane | |
| | Urethan | Letrozole | |
| | Topotecan | | |
| | | Estrogens | |
| | | Fosfestrol | |
| | | Hexestrol | |
| | | Polyestradiol Phosphate | |
| | | LHRH Analogs | |
| | | Buserelin | |
| | | Goserelin | |
| | | Leuprolide | |
| | | Triptorelin, | |
| | | | |
| | | Progestogens | |
| | | Chlormadinone Acetate | |
| | | Medroxyprogesterone | |
| | | Megestrol Acetate | |
| | | Melengestrol | |

### Cellular Vaccine Combinations

Granulocyte-macrophage colony stimulating factor (GM-CSF) is a cytokine produced by fibroblasts, endothelial cells, T cells and macrophages. This cytokine has been shown to induce the growth of hematopoetic cells of granulocyte and macrophage lineages. In addition, it activates the antigen processing and presenting function of dendritic cells, which are the major antigen presenting cells (APC) of the immune system. Results from animal model experiments have convincingly shown that GM-CSF producing tumor cells (i.e. GVAX®) are able to induce an immune response against parental, non-transduced tumor cells.

Autologous and allogeneic cancer cells that have been genetically modified to express a cytokine, e.g., GM-CSF, followed by readministration to a patient for the treatment of cancer are described in U.S. Patent Nos. 5,637,483, 5,904,920 and 6,350,445, expressly incorporated by reference herein. A form of GM-CSF-expressing genetically modified cancer cells or a "cytokine-expressing cellular vaccine" for the treatment of pancreatic cancer is described in U.S. Patent Nos. 6,033,674 and 5,985,290, expressly incorporated by reference herein. A universal immunomodulatory cytokine-expressing bystander cell line is described in U.S. Patent No. 6,464,973, expressly incorporated by reference herein. Clinical trials employing GM-CSF-expressing autologous or allogeneic cellular vaccines (GVAX®) have been undertaken for treatment of prostate cancer, melanoma, lung cancer, pancreatic cancer, renal cancer, and multiple myeloma, and a number of these trials are currently ongoing, however, the question still remains open as to whether the immune response to GM-CSF alone will be sufficiently powerful to slow or eradicate large or fast growing malignancies.

The present invention provides an improved method of stimulating an immune response to cancer in a mammalian, preferably a human, patient. Desirably, the method effects a systemic immune response, i.e., a T-cell response and/or a B-cell response, to the cancer. The method comprises administering to the patient a cytokine-expressing cellular vaccine and at least one additional cancer therapeutic agent or treatment, wherein the cellular vaccine comprises cells which express a cancer antigen or various cancer antigens, the cancer antigen/antigens can be one of the antigens of the cancer found in the patient under treatment. The cells are rendered proliferation incompetent, such as by irradiation. Upon administration of the composition, an immune response to the cancer is elicited or enhanced. In one approach, the cytokine-expressing cellular vaccine combination comprises a single population of cells that is modified to express a cytokine and at least one additional cancer therapeutic agent. In another approach, the vaccine comprises two or more populations of cells individually modified to express one component of the vaccine. In yet another approach, the cytokine-expressing cellular vaccine combination comprises a population of cells that is modified to express a cytokine which is administered in combination with at least one additional cancer therapeutic agent or treatment.

In general, a cytokine-expressing cellular vaccine combination for use in practicing the invention comprises tumor cells selected from the group consisting of autologous tumor cells, allogeneic tumor cells and tumor cell lines (i.e., bystander cells).

In some embodiments, the cells of the cytokine-expressing cellular vaccine combination are cryopreserved prior to administration. In one aspect of the invention, the cells of the cytokine-expressing cellular vaccine combination are administered to the same individual from whom they were derived (autologous). In another aspect of the invention, the cells of the cytokine-expressing cellular vaccine combination and the tumor are derived from different individuals (allogeneic or bystander). In a preferred approach, the tumor being treated is selected from the group consisting of cancer of the bladder, breast, colon, kidney, liver, lung, ovary, cervix, pancreas, rectum, prostate, stomach, epidermis; a hematopoietic tumor of lymphoid or myeloid lineage; a tumor of mesenchymal origin such as a fibrosarcoma or rhabdomyosarcoma; other tumor types such as melanoma, teratocarcinoma, neuroblastoma, glioma, adenocarcinoma and non-small lung cell carcinoma.

In one aspect of the invention, the cells of the cytokine-expressing cellular vaccine combination comprises gene-modified cells of one type for the expression of the cytokine and of another different type for expression of the one or more additional cancer therapeutic agents. By way of example, in one approach, the cytokine-expressing cellular vaccine (i.e., GVAX®) is provided as an allogeneic or bystander cell line while the one or more additional cancer therapeutic agents is expressed by autologous cells. In another approach, the one or more additional cancer therapeutic agents is expressed by an allogeneic or bystander cell line while the cytokine (i.e., GM-CSF) is expressed by autologous cells.

In previous studies, a direct comparison of murine tumor cells transduced with various cytokines demonstrated that GM-CSF-secreting tumor cells induced the best overall anti-tumor protection. In one preferred embodiment, the cytokine expressed by the cytokine-expressing cellular vaccine of the invention is GM-CSF (generally referred to herein as "GVAX®"). The preferred coding sequence for GM-CSF is the genomic sequence described in Huebner K. et al., Science 230(4731):1282-5,1985. Alternatively the cDNA form of GM-CSF finds utility in practicing the invention (Cantrell et al., Proc. Natl. Acad. Sci., 82, 6250-6254, 1985).

Prior to administration, the cells of a cytokine-expressing cellular vaccine combination of the invention are rendered proliferation incompetent. While a number of means of rendering cells proliferation incompetent are known, irradiation is the preferred method. Preferably, the cytokine-expressing cellular vaccine combination is irradiated at a dose of from about 50 to about 200 rads/min, even more preferably, from about 120 to about 140 rads/min prior to administration to the patient. Most importantly, the cells are irradiated with a total radiation dose sufficient to inhibit growth of substantially 100% of the cells, from further proliferation. Thus, desirably the cells are irradiated with a total dose of from about 10,000 to 20,000 rads, optimally, with about 15,000 rads.

### Autologous

The use of autologous cytokine-expressing cells in a vaccine of the invention provides advantages since each patient's tumor expresses a unique set of tumor antigens that can differ from those found on histologically-similar, MHC-matched tumor cells from another patient. See, e.g., Kawakami et al., J. Immunol., 148, 638-643 (1992); Darrow et al., J. Immunol., 142, 3329-3335 (1989); and Hom et al., J. Immunother., 10, 153-164 (1991). In contrast, MHC-matched tumor cells provide the advantage that the patient need not be taken to surgery to obtain a sample of their tumor for vaccine production.

In one preferred aspect, the present invention comprises a method of treating cancer by carrying out the steps of: (a) obtaining tumor cells from a mammal, preferably a human, harboring a tumor; (b) modifying the tumor cells to render them capable of producing a cytokine or an increased level of a cytokine naturally produced by the cells and at least one additional cancer therapeutic agent relative to unmodified tumor cells; (c) rendering the modified tumor cells proliferation incompetent; and (d) readministering the modified tumor cells to the mammal from which the tumor cells were obtained or to a mammal with the same MHC type as the mammal from which the tumor cells were obtained. The administered tumor cells are autologous or MHC-matched to the host.

The same autologous tumor cells may express both a cytokine and cancer therapeutic agent(s) or a cytokine and one or more cancer therapeutic agent(s) may be expressed by a different autologous tumor cell population. In one aspect of the invention, an autologous tumor cell is modified by introduction of a vector comprising a nucleic acid sequence encoding a cytokine, operably linked to a promoter and expression/control sequences necessary for expression thereof. In another aspect, the same autologous tumor cell is modified by introduction of a vector comprising a nucleic acid sequence encoding at least one additional cancer therapeutic agent operably linked to a promoter and expression/control sequences necessary for expression thereof. In a further aspect, a second autologous tumor cell is modified by introduction of a vector comprising a nucleic acid sequence encoding at least one additional cancer therapeutic agent operably linked to a promoter and expression/control sequences necessary for expression thereof. The nucleic acid sequence encoding the cytokine and additional cancer therapeutic agent(s) may be introduced into the same or a different autologous tumor cell using the same or a different vector. The nucleic acid sequence encoding the cytokine or cancer therapeutic agent may or may not further comprise a selectable marker sequence operably linked to a promoter.

### Allogeneic

Researchers have sought alternatives to autologous and MHC-matched cells as tumor vaccines, as reviewed by Jaffee et al., Seminars in Oncology, 22, 81-91 (1995). Early tumor vaccine strategies were based on the understanding that the vaccinating tumor cells function as the antigen presenting cells (APCs) and present tumor antigens by way of their MHC class I and II molecules, and directly activate the T cell arm of the immune system. The results of Huang et al. (Science, 264, 961-965, 1994), indicate that professional APCs of the host rather than the vaccinating tumor cells prime the T cell arm of the immune system by secreting cytokine(s) such as GM-CSF such that bone marrow-derived APCs are recruited to the region of the tumor. The bone marrow-derived APCs take up the whole cellular protein of the tumor for processing, and then present the antigenic peptide(s) on their MHC class I and II molecules, thereby priming both the CD4+ and the CD8+ T cell arms of the immune system, resulting in a systemic tumor-specific anti-tumor immune response. These results suggest that it may not be necessary or optimal to use autologous or MHC-matched tumor cells in order to elicit an anti-cancer immune response and that the transfer of allogeneic MHC genes (from a genetically dissimilar individual of the same species) can enhance tumor immunogenicity. More specifically, in certain cases, the rejection of tumors expressing allogeneic MHC class I molecules resulted in enhanced systemic immune responses against subsequent challenge with the unmodified parental tumor, as reviewed in Jaffee et al., supra, and Huang et al., supra.

As described herein, a "tumor cell line" comprises cells that were initially derived from a tumor. Such cells typically are transformed (i.e., exhibit indefinite growth in culture).

In one preferred aspect, the invention provides a method for treating cancer by carrying out the steps of: (a) obtaining a tumor cell line; (b) modifying the tumor cell line to render the cells capable of producing an increased level of a cytokine alone or in combination with at least one additional cancer therapeutic agent relative to the unmodified tumor cell line; (c) rendering the modified tumor cell line proliferation incompetent; and (d) administering the tumor cell line to a mammalian host having at least one tumor that is the same type of tumor as that from which the tumor cell line was obtained or wherein the tumor cell lien and host tumor express at least one common antigen. The administered tumor cell line is allogeneic and is not MHC-matched to the host. Such allogeneic lines provide the advantage that they can be prepared in advance, characterized, aliquoted in vials containing known numbers of cytokine-expressing cells and stored such that well characterize cells are available for administration to the patient. Methods for the production of gene-modified allogeneic cells are described for example in WO 00/72686A1, expressly incorporated by reference herein.

In one approach to preparing a cytokine-expressing cellular vaccine comprising gene-modified allogeneic cells, cytokine and cancer therapeutic agent-encoding nucleic acid sequences are introduced into a cell line that is an allogeneic tumor cell line (i.e., derived from an individual other than the individual being treated). In another approach, cytokine and cancer therapeutic agent encoding nucleic acid sequences are introduced into separate (i.e. different) allogeneic tumor cell lines. The cell or population of cells may be from a tumor cell line of the same type as the tumor or cancer being treated. The tumor and/or tumor cell line may be from any form of cancer, including, but not limited to, carcinoma of the bladder, breast, colon, kidney, liver, lung, ovary, cervix, pancreas, rectum, prostate, stomach, epidermis; a hematopoietic tumor of lymphoid or myeloid lineage; a tumor of mesenchymal origin such as a fibrosarcoma or rhabdomyosarcoma; or another tumor, including a melanoma, teratocarcinoma, neuroblastoma, glioma, adenocarcinoma and non-small lung cell carcinoma.

In one aspect of the invention, the allogeneic tumor cell is modified by introduction of a vector comprising a nucleic acid sequence encoding a cytokine, operably linked to a promoter and expression control sequences necessary for expression thereof. In another aspect, the same allogeneic tumor cell or a second allogeneic tumor cell is modified by introduction of a vector comprising a nucleic acid sequence encoding at least one additional cancer therapeutic agent operably linked to a promoter and expression control sequences necessary for expression thereof. The nucleic acid sequence encoding the cytokine and additional cancer therapeutic agent(s) may be introduced into the same or a different allogeneic tumor cell using the same or a different vector. The nucleic acid sequence encoding the cytokine or cancer therapeutic agent may or may not further comprise a selectable marker sequence operably linked to a promoter.

Desirably, the allogeneic cell line expresses GM-CSF in a range from 200-1000ng/10⁶ cells/24h. Preferably, the universal bystander cell line expresses at least about 200 ng GM-CSF/10⁶ cells/24 hours.

In practicing the invention, one or more allogeneic cell lines are incubated with an autologous cancer antigen, e.g., an autologous tumor cell (which together comprise an allogeneic cell line composition), then the allogeneic cell line composition is administered to the patient. Typically, the cancer antigen is provided by (on) a cell of the cancer to be treated, i.e., an autologous cancer cell. In such cases, the composition is rendered proliferation-incompetent by irradiation, wherein the allogeneic cells and cancer cells are plated in a tissue culture plate and irradiated at room temperature using a Cs source, as detailed above. The ratio of allogeneic cells to autologous cancer cells in a given administration will vary dependent upon the combination.

Any suitable route of administration can be used to introduce an allogeneic cell line composition into the patient, preferably, the composition is administered subcutaneously or intratumorally.

The use of allogeneic cell lines in practicing present invention provides the therapeutic advantage that, through administration of a cytokine-expressing allogeneic cell line and at least one additional cancer therapeutic agent (expressed by the same or a different cell line) to a patient with cancer, together with an autologous cancer antigen, paracrine production of an immunomodulatory cytokine, results in an effective immune response to a tumor. This obviates the need to culture and transduce autologous tumor cells for each patient, eliminating the problem of variable and inefficient transduction efficiencies.

### Bystander

In one further aspect, the present invention provides a universal immunomodulatory cytokine-expressing bystander cell line and a bystander cell line that expresses at least one additional cancer therapeutic agent. The same universal bystander cell line may express both a cytokine and cancer therapeutic agent or each may be expressed by a different universal bystander cell line. The universal bystander cell line comprises cells which either naturally lack major histocompatibility class I (MHC-I) antigens and major histocompatibility class II (MHC-II) antigens or have been modified so that they lack MHC-I antigens and MHC-II antigens. In one aspect of the invention, a universal bystander cell line is modified by introduction of a vector comprising a nucleic acid sequence encoding a cytokine operably linked to a promoter and expression control sequences necessary for expression thereof. In another aspect, the same universal bystander cell line or a second universal bystander cell line is modified by introduction of a vector comprising a nucleic acid sequence encoding at least one additional cancer therapeutic agent operably linked to a promoter and expression control sequences necessary for expression thereof. The nucleic acid sequence encoding the cytokine and additional cancer therapeutic agent(s) may be introduced into the same or a different universal bystander cell line using the same or a different vector. In some cases, the bystander approach is combined with the autologous or allogeneic approach. For example, an autologous, allogeneic or bystander cell line encoding a cytokine may be combined with an autologous, allogeneic or bystander cell line encoding one or more cancer therapeutic agent(s). The nucleic acid sequence encoding the cytokine or cancer therapeutic agent may or may not further comprise a selectable marker sequence operably linked to a promoter. Any combination of cytokine(s) and cancer therapeutic agent(s) that stimulate an anti-tumor immune response finds utility in the practice of the present invention. The universal bystander cell line preferably grows in defined, i.e., serum-free, medium, preferably as a suspension.

An example of a preferred universal bystander cell line is K562 (ATCC CCL- 243; Lozzio et al., Blood 45(3): 321-334 (1975); Klein et al., Int. J. Cancer 18: 421-431 (1976)). A detailed description of human bystander cell lines is described for example in U.S. Pat. No. 6,464,973 and WO9938954. Desirably, the universal bystander cell line expresses the cytokine, e.g., GM-CSF in the range from 200-1000ng/10⁶ cells/24h. Preferably, the universal bystander cell line expresses at least about 200 ng GM-CSF/10⁶ cells/24 hours.

In practicing the invention, the one or more universal bystander cell lines are incubated with an autologous cancer antigen, e.g., an autologous tumor cell (which together comprise a universal bystander cell line composition), then the universal bystander cell line composition is administered to the patient. Any suitable route of administration can be used to introduce a universal bystander cell line composition into the patient. Preferably, the composition is administered subcutaneously or intratumorally.

Typically, the cancer antigen is provided by (on) a cell of the cancer to be treated, i.e., an autologous cancer cell. In such cases, the composition is rendered proliferation-incompetent by irradiation, wherein the bystander cells and cancer cells are plated in a tissue culture plate and irradiated at room temperature using a Cs source, as detailed above.

The ratio of bystander cells to autologous cancer cells in a given administration will vary dependent upon the combination. With respect to GM-CSF-producing bystander cells, the ratio of bystander cells to autologous cancer cells in a given administration should be such that at least 36 ng GM-CSF/10⁶ cells/24 hrs is produced. In general, the therapeutic effect is decreased if the concentration of GM-CSF is less than this. In addition to the GM-CSF threshold, the ratio of bystander cells to autologous cancer cells should not be greater than 1:1. Appropriate ratios of bystander cells to tumor cells or tumor antigens can be determined using routine methods in the art.

The use of bystander cell lines in practicing present invention provides the therapeutic advantage that, through administration of a cytokine-expressing bystander cell line and at least one additional cancer therapeutic agent (expressed by the same or a different cell line) to a patient with cancer, together with an autologous cancer antigen, paracrine production of an immunomodulatory cytokine, results in an effective immune response to a tumor. This obviates the need to culture and transduce autologous tumor cells for each patient, eliminating the problem of variable and inefficient transduction efficiencies.

### Evaluation Of Combinations In Animal Models

### B16F10 melanoma Model

In one approach, the efficacy of cytokine-expressing cellular vaccine combination is evaluated by carrying out animal studies in the syngeneic B16F10 melanoma tumor model in the treatment setting. See, e.g., Griswold DP Jr., Cancer Chemother Rep 2;3(1):315-24, 1972 and Berkelhammer J et al., Cancer Res 42(8):3157-63, 1982. The murine melanoma cell line B16 is a well-defined cell line which is weakly immunogenic in syngeneic C57BL6 mice and therefore readily forms tumors in C57BL6 mice.
Furthermore, several tumor associated antigens have been identified in this model which allow one to monitor tumor as well as antigen specific immune responses. In addition, several murine-specific reagents are commercially available and are used to monitor anti-tumor immune responses in the various vaccine strategies. A typical study in the B16F10 melanoma tumor model makes use of at least 6 and generally 10-15 mice per group in order to obtain statistically significant results. Statistical significance is evaluated using the Student's t- test.

Vaccination of C57BL/6 mice with irradiated GM-CSF-secreting B16F10 tumor cells stimulates potent, long-lasting and specific anti-tumor immunity that prevents tumor growth in most mice subsequently challenged with wild-type B16F10 cells. However, this protection is less effective when GM-CSF-producing tumor cell vaccines are administered to mice with preexisting tumor burden. In carrying out studies using the B16F10 melanoma tumor model, female C57BL/6 mice are obtained from Taconic and are 6-8 weeks old at the start of each experiment. In a typical experiment, mice are injected with 1x10⁵ B16BF10 cells on day 0 subcutaneously in a dorsal/anterior location. On day 3, mice are vaccinated in a ventral/posterior location with 1-3x10⁶ irradiated (5000 rads) B16F10 or cytokine-expressing cellular vaccine (e.g., GVAX®). Mice are followed for tumor development and survival. After 14-21 days, mice are sacrificed and their tumor burden assessed by harvesting the mice lungs and counting the surface tumor metastasis and measuring the weight of the lung. An alternative B16F10 melanoma tumor model involves subcutaneous injection of B16F10 tumor cells.

A typical in vivo study in the B16F10 melanoma tumor model employs the following groups: HBSS only (negative control); irradiated B16F10/HBSS (control): cytokine-expressing cellular vaccine (GVAX®)/HBSS; (cellular vaccine monotherapy control); additional cancer therapeutic agent or treatment (therapeutic agent/treatment monotherapy control); additional cancer therapeutic agent or treatment together with tumor antigen source; cytokine-expressing cellular vaccine plus cancer therapeutic agent or treatment.

Experiments in the syngeneic B16 melanoma model in C57BL6 mice, have shown that immunity was induced with B16 cells that were genetically modified to express GM-CSF, while non-transduced B16 cell were completely ineffective. Vaccination of C57BL6 mice with irradiated B16F10 melanoma cells engineered to secrete GM-CSF has been shown to stimulate potent, long-lasting and specific anti-tumor immunity that prevents tumor formation in a majority of mice challenged with non-transduced B16F10 (prevention model; Fig 1A). However, when irradiated GM-CSF-producing tumor cell are administered to mice harboring recently established subcutaneous tumors (treatment model; Fig 1B), the protective anti-tumor immunity is less effective. Results from animal model experiments have convincingly shown that GM-CSF producing tumor cells are able to induce an immune response against the parental, non-transduced tumor cells, even if they are non-immunogenic tumor cells, such as B16F10.

Previous experiments have demonstrated that HBSS or irradiated B16F10 alone do not protect challenged mice from tumor formation. GM-CSF-expressing cellular vaccines (GVAX®) alone were shown to protect from 30-50% of the challenged mice. The combination of a cytokine-expressing cellular vaccine plus at least one additional cancer therapeutic agent or treatment is expected to increase the efficacy of anti-tumor protection. The degree of protection depends on several factors such as the expression level of the additional cancer therapeutic agent and the cytokine-expressing cellular vaccine, the level of treatment (i.e. dose of the agent or the frequency and strength of radiation) and the relative timing and route of administration of the additional cancer therapeutic agent (e.g., as transfected cells or as a protein or chemical entity) relative to the timing of administration of the cytokine-expressing cellular vaccine, e.g., GVAX®.

### Immunological monitoring

Several tumor associated antigens have been identified which allow one to monitor tumor as well as antigen specific immune responses. For example, tumor antigen-specific T cells can be identified by the release of IFN-gamma following antigenic restimulation in vitro (Hu, H-M. et al., Cancer Research, 2002, 62; 3914-3919). Yet another example of new methods used to identify tumor antigen-specific T cells is the development of soluble MHC I molecules also known as MHC tetramers (Beckman Coulter, Immunomics), reported to be loaded with specific peptides shown to be involved in an anti-tumor immune response. Examples within the B16F10 melanoma tumor model include but are not limited to gp100, Trp2, Trp-1, and tyrosinase. Similar melanoma-associated antigens have been identified in humans. Such tools provide information that can then be translated into the clinical arena.

### B16.OVA Model

B16.ova and B16.GM.ova tumors are B16 cells or B16 cells expressing GM-CSF that were modified to express membrane bound ovalbumin. Ovalbumin acts as a surrogate tumor associated antigen on the tumor cells used for challenge as well as on the vaccine cells. Ovalbumin-specific T cells are used to track "tumor specific" T cell responses in the presence or absence of cells expressing GM-CSF (B16.GM-ova) alone or in combination with another cancer therapeutic agent. An antibody specifically recognizing the T-cell receptor of the ovalbumin specific T-cells is used to follow these "tumor-specific" T-cells. This antibody can be used to monitor the expansion of these tumor-specific T-cells and their activation status following various vaccination regiments. In one exemplary experimental approach, mice received via adoptive transfer ovalbumin-specific T-cells on day -2, are challenged with B16F10.ova on day 0, are vaccinated with B16F10.GM-ova on day 3 and then treated with at least one additional cancer therapeutic agent such as taxotere on day 5 and 9 (6 mg/kg), followed by monitoring of OVA-specific T cells at various time points after vaccination.

### RIP- Tag Spontaneous Pancreatic Islet Carcinoma Model

The RIP-Tag spontaneous pancreatic islet carcinoma model makes use of transgenic mice which have been genetically modified to express a rat insulin promoter (RIP) driven simian virus 40 (SV-40) antigen and develop islet cell carcinomas as a result of SV-40 oncogeneA expression in pancreatic islet cells. In the model, tumor development proceeds through a series of well-defined stages that occur over a period of 13.5 weeks in these mice. The RIP-Tag mice develop pancreatic insulinomas and islet cell carcinomas in a multi-step pathway that includes an angiogenic switch before solid tumor formation. 100% of the normal islets express the Tag oncogene however, do not show symptoms of dysplasia until 3-4 weeks of age. Hyperplastic cells, 50% of the islets, begin to appear by 10 weeks of age. Solid tumors emerge after 12-13 weeks that progress into large adenomas that sometimes develop into invasive carcinomas, characterized by high vasculature and dilated hemorrhagic vessels. (Bergers G et al., Science, 1999 Apr 30;284(5415):808-12). The RIP-Tag spontaneous pancreatic islet carcinoma model may be used to evaluate the efficacy of cytokine-expressing cellular vaccine combinations.

### Assays For Efficacy Of Combinations In Vivo Models

Tumor burden is assessed at various time points after tumor challenge. Typically, spleens cells are assessed for CTL activity by in vitro whole cell stimulation for 5 days. Target cells are labeled with ⁵¹Cr and co-incubated with splenic effector CTL and release of ⁵¹Cr into the supernatants as an indicator of CTL lysis of target cells. On day 3 of in vitro stimulated CTL supernatants are tested for IFN-gamma production by CTL. In brief, wells are coated with coating antibody specific for IFN-gamma, supernatant is then added to wells, and IFN-gamma is detected using an IFN-gamma specific detecting antibody. IFN-gamma can also be detected by flow cytometry, in order to measure cell-specific IFN-gamma production.

Another indication of an effective anti-tumor immune response is the production of effector cytokines such as TNF-alpha, IL-2, and IFN-gamma upon restimulation in vitro. Cytokine levels were measured in supernatants from spleen cells or draining lymph node (dLN) cells restimulated in vitro for 48 hours with irradiated GM-CSF-expressing cells.

A further method used to monitor tumor-specific T cell responses is via intracellular cytokine staining (ICS). ICS can be used to monitor tumor-specific T-cell responses and to identify very low frequencies of antigen-specific T-cells, Because ICS is performed on freshly isolated lymphocytes within 5 hours of removal, unlike the CTL and cytokine release assays, which often require 2-7 days of in vitro stimulation, it can be used to estimate the frequency of tumor antigen-specific T-cells in vivo. This provides a powerful technique to compare the potency of different tumor vaccine strategies. ICS has been used to monitor T-cell responses to melanoma-associated antigens such as gp100 and Trp2 following various melanoma vaccine strategies. Such T-cells can be identified by the induction of intracellular IFN-gamma expression following stimulation with a tumor-specific peptide bound to MHC I.

### Xenogen Imaging Of Tumor Models

In some studies, in vivo luminescence of tumor bearing mice is monitored by monitoring of B16F10-luciferase (Xenogen Inc.) injected mice. In brief, Balb/c nu/nu mice are injected with 5 x 10⁴ or 2x 10⁵ cells of B16F10-luc cells via tail vein on day 0. Mice are monitored for tumor burden when necessary by intra-peritoneal injection of excess luciferin substrate at 1.5 mg/g mice weight. In a typical analysis, twenty minutes after substrate injection, mice are anesthesized and monitored for in vivo luminescence with Xenogen IVIS Imaging System (Xenogen Inc.) luminescence sensitive CCD camera by dorsal or ventral position. Data is collected and analyzed by Living Image 2.11 software.

### Cytokine-Expressing Cellular Vaccine Combinations

The present invention is directed to combinations of a cytokine-expressing cellular vaccine (e.g., GVAX®) plus at least one additional cancer therapeutic agent or treatment. The agent or treatment may be a chemotherapeutic agent, an agent that modulates the immune response to a cancer antigen, radiation, etc. Exemplary embodiments of the invention, include, but are not limited to a cytokine-expressing cellular vaccine plus one or more of the following: an anti-CTLA-4 monoclonal antibody; an anti-41BB mAb; anti-CD40 or membrane/soluble CD40L; interferon alpha (IFN-alpha); IL-2 or IL-18; a HSP (heat shock protein); CpG (dinucleotides that mimic bacterial DNA); anti-OX-40 or soluble/membrane bound OX-40 Ligand; Cox-2 inhibitors; Iressa; Imiquimod; Juvaimmune; anti-epidermal growth factor R (EGF-R); anti-VEGF R or TRAIL. Exemplary combinations are described in more detail below.

### Co-Stimulatory Molecules in Combination with Cytokine-Expressing Cellular Vaccines

In natural immune responses, CD4+ T helper (Th) cells, reactive with peptide antigens presented by MHC class II molecules on dendritic cells (DC), can drive the maturation of DC which is required for induction of CD8+ CTL immunity. Proper induction, expansion and maintenance of CTL responses are achieved through the interaction between CD4+ T cells, DC and CD8+ T cells. While the mechanism is not part of the invention, the cells to a large extent operate through up-regulation of CD40L, which interacts with DC-expressed CD40 to effect DC maturation. CD80/CD86 expressed by mature or activated DC can effect CTL induction by interaction with the CD28 costimulatory receptor on CD8+ T cells. For maintenance and full expansion of CTL, interaction of the DC-expressed 4-1BB ligand with its receptor 4-1BB on CTL is also important. DC activation may be triggered by e.g., agonistic anti-CD40 antibody or ligands of Toll-like receptors (TLR) such as LPS (TLR4 ligand) or oligodeoxynucleotides containing CpG-motifs (TLR9 ligand).

### Cytokine-Expressing Cellular Vaccines (e.g., GVAX®) plus an anti-CTLA-4 monoclonal antibody

The CTLA4 molecule, expressed at low levels on resting T cells and induced by T cell activation, transduces a negative signal to T cells which blocks IL-2 production, IL-2R expression, and induces cell cycle arrest. CTLA4 regulates both CD28 and non-CD28 mediated T cell activation, and has an important role in normal immune homeostasis (Bour-Jordan et al., Nature Immunol. 4: 182-188, 2003). In animal models, anti-CTLA-4 antibody can induce tumor rejection in immunogenic tumors, and in combination with anti-tumor vaccination, can induce rejection of minimally immunogenic tumors. Clinical trials have been initiated with a fully human antibody that binds to human CTLA-4 for treatment of metastatic melanoma and hormone refractory prostate carcinoma (HRPC) patients.

Previous reports indicate that an anti-CTLA-4 monoclonal antibody (anti-CTLA4 mAb) or GM-CSF-secreting tumor cell vaccine provides only partial protection of mice when either is used as a monotherapy for non-immunogenic tumors such as B16 melanoma. The results presented herein demonstrate that the combination of GM-CSF-secreting B16 tumor cells and anti-CTLA-4 acts synergistically, resulting in highly protective anti-tumor immune responses. In order to achieve the maximal synergistic effect of these two agents in clinical trials, it is essential to carefully evaluate possible treatment regimens in preclinical studies. In ongoing clinical trials GM-CSF-secreting tumor cell vaccines (GVAX^{®}) or human anti-CTLA4 mAb are administered to patients repeatedly over a period of several months. In studies described herein, the efficacy of the combination was evaluated in preclinical studies following repeated administration of both anti-CTLA-4 mAb and GM-CSF-secreting tumor cell vaccines. Example 1 details studies where a hamster-anti mouse anti-CTLA4 (9H10) and a murine anti-mouse anti-CTLA-4 mAb (9D9), which blocks B7/CTLA-4 interactions were tested in the B16 melanoma tumor model with and without vaccination with a cytokine-expressing cellular vaccine (GM-CSF-secreting B16F10 tumor cells; B16-GM) (Fig. 2A). Initial preclinical studies in mice used the hamster-anti mouse anti-CTLA4 (9H10) Ab, which precluded repeated administration of the anti-CTLA4 mAb due to the development of anti-hamster Abs. Therefore, the murine anti-mouse anti-CTLA-4 mAb, 9D9, was generated. The 9D9 anti-CTLA-4 mAb was ineffective as a monotherapy in protecting C57Bl/6 mice from a B16F10 tumor challenge. In contrast, 80% of the mice treated with B16-GM plus the anti-CTLA4 antibody, 9D9, were protected, demonstrating that 9D9 is biologically active and further supports the concept that anti-CTLA-4 and GM-CSF-secreting tumor cell vaccines (such as GVAX®) are an effective anti-cancer combination therapy (Figs. 2B - 2E).

These results demonstrate that in practicing the present invention an autologous, allogeneic, or bystander cytokine-expressing cellular vaccine (e.g., GVAX®) may be administered to a cancer patient in combination with an anti-CTLA-antibody resulting in enhanced therapeutic efficacy and prolonged survival relative to either monotherapy alone.

### Cytokine-Expressing Cellular Vaccines (e.g., GVAX®) plus anti-41BB mAb

CD137, also known as 4-1BB, is a member of the TNFR superfamily (Kwon BS et al., Cell Immunol. 121(2):414-22, 1989; Kwon BS et al., Proc Natl Acad Sci U S A. 86(6):1963-7, 1989). CD137 is expressed as monomers, dimers, and tetramers on the surface of activated T cells around day 4-6 after activation through the TCR complex. Interaction of CD137 with its ligand, 4-1BBL, is reported to deliver a costimulatory signal leading to expansion, cytokine production, including IFN-g, development of CTL and inhibition of activation induced cell death (AICD). 4-1BB is expressed on activated T cells with the peak of expression 2-3 days post activation. The ligand for 4-1BB (4-1BBL) is expressed on activated APC. Several studies have demonstrated that administration of anti-4-1BB monoclonal antibody can increase CTL responses and/or eradicate established tumors in vivo. See, e.g., Shuford et al. J. Exp. Med.; 1997 and Melero et al., Nature medicine; 1997.

4-1BB ligand is described in US Pat. No. 5,674,704; 4-1BB ligand polypeptides and a cell surface receptor that binds 4-1BB ligand are described in US Pat. No. 6,355,779; monoclonal antibodies (mAb) which specifically bind to the extracellular domain of 4-1BB are described in US Pat. Nos. 6,210,669 and 5,928,893; a humanized antibody specific for human 4-1BB is described in US Pat. No. 6,458,934; and a method to enhance T cell proliferation by administering an agonistic mAb which binds to 4-1BB is described in US Pat. No. 6,303,121, each of which is expressly incorporated by reference herein.

As detailed in Example 2, the efficacy of cytokine-expressing cellular vaccine/4-1BB combinations was evaluated in the B16F10 tumor model and the results show that the combination enhanced both the number of tumor free mice (Fig. 4A) and survival (Fig. 4B) and for up to 80 days post-challenge. These results demonstrate that in practicing the present invention an autologous, allogeneic, or bystander cytokine-expressing cellular vaccine (e.g., GVAX®) is administered to a cancer patient in combination with an anti 4-1BB antibody resulting in enhanced therapeutic efficacy relative to either monotherapy alone.

### Cytokine-Expressing Cellular Vaccines (e.g., GVAX®) Plus One Or More Additional Cytokines

In one aspect of the invention, a cytokine-expressing cellular vaccine (e.g., GVAX®) is administered to a patient in combination with at least one additional cytokine for the treatment of cancer. Exemplary cytokines include, but are not limited to IL-2 through IL-29, r-IFN, TNF-alpha, CD2, MIP3a and ICAM.

### Cytokine-Expressing Cellular Vaccines (e.g., GVAX®) Plus Interferon-alpha

Interferons were identified in 1957 as a factor that interferes with viral infection. Type I (IFN-α and IFN-β) are produced during the early phase of viral infection. Type II (IFN-y) is important in the development of cellular immune responses. IFN-alpha has been shown to have a direct anti-proliferative effect on tumor cells via cytotoxic effects (apoptosis). IFN-alpha is known to modulate antigen specific immunity by changing the microenvironment, which favors the proliferation of Th1 lymphocytes. IFN-α is important for the generation of CTLs, by promoting clonal expansion and survival of CD8+ T cells.

IFN-alpha is approved for the clinical treatment of lymphoma, leukemia (hairy cell), Kaposi's sarcoma, and melanoma. The effectiveness of IFN-alpha in cancer therapy may be a result of both immune-dependent and independent mechanisms. For example, IFN-alpha has been shown *in vitro* to exert immunomodulatory effects that include the upregulation of MHC class I molecules and the enhancement of dendritic cell activation/differentiation. In addition, IFN-alpha promotes the development of Th1 lymphocytes and tumor-antigen specific CTL and acts as a survival factor for activated CD8⁺ T cells both *in vitro* and *in vivo.* Immune independent processes may include anti-proliferative and/or pro-apoptotic activity. All of these properties would be critical for the development of effective anti-tumor responses.

Therapeutic strategies that target tumor angiogenesis have recently emerged as potent options for the treatment of cancer. Clinical evidence suggests that IFN-alpha may also inhibit tumor growth by anti-angiogenic processes. The combination of IFN-alpha and a GM-CSF-secreting tumor cell vaccine was investigated in the B16F10 melanoma mouse model (as described in Example 3).

Studies were undertaken to investigate the effect of the combination of a cytokine-expressing cellular vaccine (GM-CSF-secreting B16F10 tumor cells; B16-GM) together with IFN-α. Recombinant murine IFN-α (rmIFN-alpha) given as a monotherapy protected up to 30% of mice challenged with B16F10 tumor cells when it was administered subcutaneously at the tumor challenge site, but not when administered at a distant site. Similarly, 30-40% of animals that received B16-GM alone remained tumor free after challenge. In contrast, administration of the combination of IFN-alpha and B16.GM resulted in 70-80% tumor free survival (Example 3). Furthermore, IFN-alpha can also be administered at the vaccine site resulting in an increase of vaccine potency from 10% (vaccine only) to 50-60% (vaccine + IFN-alpha). In summary, the results demonstrate that IFN-alpha combined with a GM-CSF secreting cellular vaccine can significantly delay tumor growth in the B16F10 model, suggesting that the combination represents an effective strategy for therapy for cancer.

In one preferred embodiment of the invention, the additional cancer therapeutic agent is interferon-alpha, which is administered together with a GM-CSF-expressing cellular vaccine. In practicing the present invention, interferon-alpha may be administered in the form of the protein or in the form of an autologous, allogeneic, or bystander interferon-alpha-expressing cell line.

### Cytokine-Expressing Cellular Vaccines (e.g., GVAX®) Plus IL-2

Previous studies have shown that transfection of a gene encoding IL-2 into a tumor cell stimulated an MHC class I-restricted cytolytic T lymphocyte (CTL) response against the tumor in vivo, suggesting that IL-2 can play a role in enhancing the immune responsiveness to a tumor in vivo (Frost et al., WO 92/05262; Fearon et al., Cell 1990 Feb 9;60(3):397-403). An exemplary IL-2 sequence for use in practicing the invention may be found for example at GenBank Accession No. NMIL04, NM_000586, expressly incorporated by reference herein. Previous studies in the B16 melanoma model have shown that cells expressing both IL-2 and GM-CSF can generate systemic immunity and enhanced survival, as described in WO 00/72686, expressly incorporated by reference herein.

The efficacy of a cytokine-expressing cellular vaccine/IL-2 combination has been previously evaluated in the B16F10 tumor model (as described for example in WO 00/72686). In practicing the present invention an autologous, allogeneic, or bystander cytokine-expressing cellular vaccine (e.g., GVAX®) is administered to a cancer patient in combination with IL-2 protein or as an autologous, allogeneic, or bystander IL-2-expressing cell line.

### Cytokine-Expressing Cellular Vaccines (e.g., GVAX®) plus Chemotherapeutic agents

Embodiments of the present invention include the administration of the combination of a cytokine-expressing cellular vaccine and at least one additional traditional cancer therapeutic agent. Traditional cancer therapeutic agents for use in practicing the invention include those listed in Tables 1A and 1B. These include agents from each of the major classes of cancer therapeutic agents, including but not limited to: alkylating agents, alkaloids, antimetabolites, anti-tumor antibiotics, nitrosoureas, hormonal agonists/antagonists and analogs, immunomodulators, photosensitizers, enzymes and others.

### Cytokine-Expressing Cellular Vaccines (e.g., GVAX®) plus docetaxel (TAXOTERE™)

Docetaxel is a semi-synthetic taxane that has demonstrated therapeutic efficacy against breast, prostate, ovarian and other solid tumors. The tumoricidal activity of docetaxel is mainly attributed to its ability to block microtubule depolymerization thus inducing G₂-M arrest and apoptosis. However, mounting evidence suggests that docetaxel also possesses immunomodulatory activity such as augmenting macrophage and lymphokine activated killer activity as well as the production of pro-inflammatory cytokines. These properties make docetaxel an interesting chemotherapeutic agent to combine with other cancer immunotherapies. One of skill in the art will appreciate that paclitaxel (TAXOL™) may be used in place of docetaxel (TAXOTERE™).

The combination of docetaxel and a GM-CSF-secreting B16F10 tumor cell (B16-GM) vaccine was evaluated in the B16 melanoma model (Example 4). In summary, the results demonstrate that docetaxel combined with a GM-CSF secreting cellular vaccine can significantly delay tumor growth in the B16F10 model, suggesting that the combination represents a new strategy for combining chemo and immunotherapy for cancer. In practicing the present invention an autologous, allogeneic, or bystander cytokine-expressing cellular vaccine (e.g., GVAX®) is administered to a cancer patient in combination with docetaxel.

### Cytokine-Expressing Cellular Vaccines (e.g., GVAX®) plus Cox-2 inhibitors

Cyclooxygenase (COX) (also known as PGH synthase or prostaglandin endoperoxide synthase), is the key enzyme catalyzing the biosynthesis of Prostaglandins (PGs). There are two genes that express two distinct isoforms of the enzyme COX-1 and COX-2, which have similar primary protein structure (60% homology) and catalyze essentially the same reaction. Prostaglandins are involved in as diverse normal processes as renal function, vasomotor tone, platelet aggregation and blood clotting, differentiation of immune cells, wound healing, nerve growth, bone metabolism, ovulation, and initiation of labor. COX-2, is an inducible isoform that is found mainly in inflammatory and immune cells (neutrophils, macrophages, mast cells, etc). Pro-inflammatory cytokines and growth factors induce COX-2, which suggests that it may play an important role in the process of inflammation and in the control of cell growth. COX-2 is strongly expressed in human colon cancer cells, and is thought to delay the progress of colon tumors possibly by causing apoptosis (programmed cell death) of the tumor cells. See, e.g., Vane JR et al., Annu Rev Pharmacol Toxicol, 1998, 38:, 97-120; Cryer B and Feldman M., Am J Med, 1998 May, 104:5, 413-21; S. Dubinett et al., Clincical Cancer Research; 2003; and U. Yamashita et al., J. Immonology; 2000. Epidemiological studies as well as early clinical trials also suggest that administration of COX-2 inhibitors may reduce the risk of cancer development. To date, the FDA has approved the use of a selective COX-2 inhibitor, Celecoxib (CELEBREX ®), for the treatment of familial adenomatous polyposis (FAP) in patients. Additionally, numerous rodent models have demonstrated that COX-2 inhibitors reduce tumor formation in a number of different tumors. COX-2 inhibitors may reduce tumor formation by inhibiting tumor vascularization. Murine COX-1 is 92% homologous to human COX-1 and murine COX-2 is 91% homologous to human COX-2.

The combination of the COX-2 inhibitor, Celebrex, was evaluated in combination with B16-GM in the B16F10 model to test whether a cytokine-expressing cellular vaccine plus a selective COX-2 inhibitor led to greater protection than vaccination with either agent alone (Example 5). Preliminary results show that Celebrex alone has no effect on B16F10 tumor size and overall survival as a single agent. However, when combined with a GM-CSF-expressing cellular vaccine, a significant increase in the number of surviving and tumor free animals was observed (Figs. 9A and B). The results show that the COX-2 inhibitor must be administered before vaccination with the cytokine-expressing cellular vaccine in order for efficacy. In the B16F10 model, the best efficacy was observed when Celebrex was administered at least 3 days before vaccination and that when Celebrex was administered on the day of vaccine, no enhancement was observed. In further studies, the data also demonstrates that as a monotherapy Celebrex does have a significant effect on CT26 tumor size and overall survival of BALB/c mice (Example 5).

The results suggest that the combination of a cytokine-expressing cellular vaccine (i.e., GVAX®) and a COX-2 inhibitor leads to greater overall protection than treatment with the individual agents. In practicing the present invention an autologous, allogeneic, or bystander cytokine-expressing cellular vaccine is used in combination with a *COX-2* inhibitor.

### Cytokine-Expressing Cellular Vaccines (e.g., GVAX®) plus anti-CD40 or membrane/soluble CD40L

CD40L binds to CD40. CD40 is expressed on B cells and dendritic cells. CD40 has a central role in B-cell growth, B cell differentiation and B cell survival. Signaling thru CD40 rescues B cells from apoptosis induced by FAS. It also induces B cells to undergo Ig isotype switching and to express CD80 (B7 or B7.1). Antigen presenting cells (APCs), including specialized dendritic cells (DC), play a pivotal role in the direct activation and differentiation of T cells by providing co-stimulatory signals and cytokines involved in the development of cellular immune responses. Granulocyte-macrophage colony stimulatory factor (GM-CSF), a cytokine produced by fibroblasts, endothelial cells, T cells, and macrophages, activates the processing and antigen-presenting function of DC. Irradiated B16F10 melanoma cells transduced to express GM-CSF (B16-GM) confer partial protective anti-tumor immunity to recently established subcutaneous B16F10 tumors. Membrane-bound CD40 ligand (CD40L) and anti-CD40 monoclonal antibody have been shown to activate DCs. CD40, a member of the tumor necrosis factor receptor (TNF-R) family, is a surface receptor expressed on antigen presenting cells (APCs) including, B cells, DCs, and activated monocytes. The natural ligand for CD40 is CD40L. CD40L enhances the antigen presentation function of CD40-expressing B cells and DCs, resulting in the production of IL-12, which plays a critical role in the development of effector T cells.

Example 6 details in vivo studies directed to evaluate cytokine-expressing cellular vaccines in combination with either anti-CD40 or membrane bound CD40 ligand (CD40L) (Figs. 10 and 11). Experimental results show that B16F10 cells transduced by adenovirus to express CD40L, given in combination with B16-GM, significantly increased the survival of mice challenged with B16F10 melanoma, when administered 4 days after vaccination with B16-GM. CD40L administered the same day with B16-GM was shown to exacerbate the disease. To further demonstrate the efficacy of B16-GM and anti-CD40 combinations, animals were treated with an anti-CD40 monoclonal antibody administered at different times post vaccination. Similar to results obtained for CD40L, the timing of anti-CD40 administration with respect to vaccination was critical to efficacy. The data indicate that targeting DCs can enhance the potency of vaccination with GM-CSF secreting cellular vaccines.

The efficacy of a cytokine-expressing cellular vaccine/CD40L and anti-CD40 combinations were evaluated in the B16F10 tumor model (as further described in Example 6). In practicing the present invention an autologous, allogeneic, or bystander cytokine-expressing cellular vaccine (e.g., GVAX®) is administered to a cancer patient in combination with an anti-CD40 antibody or CD40 ligand expressed by autologous, allogeneic, or bystander cells, resulting in enhanced therapeutic efficacy relative to either monotherapy alone.

### Cytokine-Expressing Cellular Vaccines (e.g., GVAX) plus anti-OX40 mAb or membrane/soluble OX40L.

OX40 (CD134), a member of the TNFR superfamily, is expressed on activated Cb4+ T cells. Crosslinking OX40 on the surface of CD4 T cells generates a potent costimulatory signal that enhances T -cell proliferation to submitogenic concentrations of Con A, anti- CD3, PHA and PMA. Antigen-specific T -cell responses can also be enhanced by OX40-mediated costimulation. OX40 engagement in vivo inhibits the peripheral deletion of CD4+ T cells that occurs after their expansion in response to superantigens or soluble protein Ag, and OX40 knockout mice are deficient in generating normal levels of Ag- specific memory T cells following immunization in vivo. Thus, OX40 engagement appears to be important for the generation and maintenance of memory T cells following in vivo immunization. The combination of GM-CSF-producing tumor cells and stimulation through the OX40 receptor (OX40R) should provide an enhanced therapeutic effect towards cancer than the individual therapies. In order to optimize the clinical efficacy of a cytokine-expressing cellular vaccine/OX40L or anti-OX40 combination, a number of treatment regimens are evaluated in syngeneic tumor models (as further described in Example 7 and Gri, G. et al. J. Immunol. 2003, Jan 1 ;170(1):99-106). In practicing the present invention an autologous, allogeneic, or bystander cytokine-expressing cellular vaccine (e.g., GVAX®) is administered to a cancer patient in combination with either anti-OX40 antibody or as an autologous, allogeneic, or bystander OX40L-expressing cell line.

### Cytokine-Expressing Cellular Vaccines (e.g., GVAX®) plus Anti-angiogenic agents

It is generally accepted that tumor development requires the secretion by cancer cells of soluble mediators, so-called "tumor angiogenic factors", which activate the formation of new blood vessels. The discovery of vascular endothelial growth factor (VEGF) and of new proteases had led to the identification of compounds involved in tumor angiogenesis and has allowed for the design of new therapeutic strategies, including the use of anti-angiogenic compounds.

In one aspect, the invention provides a cytokine-expressing cellular vaccine combination comprising an anti-angiogenic compound. Preferred anti-angiogenic compounds include endostatin, angiostatin, platelet factor-4, the 16-kD fragment of prolactin, sFlt-1 (a soluble fragment of fms-like tyrosine kinase 1 receptor), sKDR (soluble fragment of kinase insert domain receptor), VEGF-Trap (hybrid soluble receptor consisting of domains from VEGF receptor 1 and VEGF receptor 2), sVEGFR3 (soluble extracellular forms of VEGFRs), anti-epidermal growth factor R (EGF-R), TRAIL (tumor necrosis factor - related apoptosis-inducing ligand), thrombospondin, interferon alpha and (PEDF) pigment epithelium-derived factor, as well as factors involved in signaling pathways that lead to expression/production of these compounds.

In practicing the present invention an autologous, allogeneic, or bystander cytokine-expressing cellular vaccine (e.g., GVAX®) is administered to a cancer patient in combination an anti-angiogenic agent delivered in the form of a protein or as an autologous, allogeneic, or bystander anti-angiogenic agent-expressing cell line.

### Cytokine-Expressing Cellular Vaccines (e.g., GVAX®) plus HSP (heat shock protein)

Recent evidence suggests that heat shock proteins (HSPs) associate with a broad array of self- peptides that can be presented on MHC I molecules through a mechanism called cross-presentation. Additionally, HSPs may activate professional APC through specific receptor interactions that stimulate pro inflammatory cytokines and upregulation of costimulatory molecules. As a result tumor derived HSPs are potent stimulators of tumor-specific immune responses.

The efficacy of a cytokine-expressing cellular vaccine/HSP combinations are evaluated in the B16F10 tumor model (as further described in Example 8). An exemplary HSP sequence for use in practicing the invention may be found for example at GenBank Accession No. AX194370, expressly incorporated by reference herein.

In practicing the present invention an autologous, allogeneic, or bystander cytokine-expressing cellular vaccine (e.g., GVAX®) is administered to a cancer patient in combination with HSP or as an autologous, allogeneic, or bystander HSP-expressing cell line.

### Cytokine-Expressing Cellular Vaccines (e.g., GVAX®) plus CpG

Mammalian DNA contains CG- at a low frequency and even when present, the 5-position of cytosine is methylated so that it is not recognized by the mammalian immune system, In contrast, bacterial DNA contains a large number of unmethylated CG-dinucleotides which are recognized by the vertebrate immune system. CpG ODN have been shown to exhibit potent immunostimulatory properties that enhance the function of both the innate and adaptive immune response. Unmethylated cytosine-phosphorothioate-gaunine (CpG) motifs prevalent in bacterial DNA and many viral DNAs, can activate B cells, DCs, monocytes, and NK cells to produce a variety of cytokines that have potent adjuvant effects. CpG oligos are known to facilitate enhanced stimulation of Th1 cytokine production, activation of antigen presenting cells such as dendritic cells and macrophages, and induce increased IFN-α production, thereby promoting a variety of anti-cancer immune effects. The combination of a CpG adjuvant with a 35 amino acid long synthetic peptide comprising both tumor-specific CTL and Th epitopes proved to be a highly effective vaccine formulation capable of inducing therapeutic immunity against human papillomavirus-induced mouse tumors (Kim et al. Cancer Res. 62(24):7234-40. 2002).

Synthesized CpG motifs have been evaluated in combination with B16-GM in the B16F10 model to test whether a cytokine-expressing cellular vaccine plus CpGs will lead to greater protection than vaccination with either agent alone. Preliminary results show that certain CpGs administered IP can enhance the effects of GM-CSF primed T cells and that certain CpGs administered before B16-GM vaccination have a greater synergistic effect than if administered after vaccination.

The results suggest that the combination of a cytokine-expressing cellular vaccine (i.e., GVAX®) and CpGs can lead to greater overall protection than the individual therapies. In practicing the present invention an autologous, allogeneic, or bystander cytokine-expressing cellular vaccine (e.g., GVAX®) is administered to a cancer patient in combination with a CpG dinucleotide.

### Cytokine-Expressing Cellular Vaccines (e.g., GVAX®) + Juvaimmune

Cationic lipid-DNA complexes (JuvImmune) are very potent at triggering immune activation and interferon release. The immunological responses elicited by JuvImmune have been successfully demonstrated in a variety of mammals including mice, rats, rabbits, cats, dogs, goats, and monkeys. The synergistic combination of lipid and non-coding DNA, with or without disease-specific antigens, provides a potent immunological stimulus, which activates substantial Th1-mediated response (production of IFN-alpha, IFN-gamma and IL-12). JuvImmune and a cytokine-expressing cellular vaccine such as GVAX® may find utility in the treatment of cancer.

### Cytokine-Expressing Cellular Vaccines (e,g., GVAX®) + Imiquimod

Imiquimod, a novel synthetic molecule developed by 3M Pharmaceuticals, is one of the best pharmacological agents that modulate the functions of dendritic cells (DCs) and macrophages. Imiquimod has been evaluated as a topical therapy for genital warts and also showed efficacy in clinical trials as a topical therapy for basal cell carcinoma. Imiquimod modulates immune responses by activating DCs, macrophages and other cells via Toll receptor 7 (TLR-7) binding and has been shown to induce the synthesis of cytokines including IFN-alpha, IL-1, IL-6, IL-8, IL-10, IL-12, TNF- alpha, and GM-CSF. The combination of Imiquimod and a cytokine-expressing cellular vaccine such as GVAX® may find utility in the treatment of cancer.

### Cytokine-Expressing Cellular Vaccines (e,g., GVAX®) + Iressa

Iressa is clinically accepted as a third line chemotherapy drug for patients with Non Small Cell Lung Cancer (NSCLC). It is a small molecule that inhibits the intracellular phosphorylation of EGF receptor TK, which interferes with downstream signal transduction involved in proliferation, angiogenesis, metastasis, and resistance to apoptosis, thereby leading to cell death. The anti-angiogenic activity of Iressa will slow/inhibit the growth of a tumor, which will allow cytokine-expressing cellular vaccine-induced tumor-specific immune response to destroy the remaining tumor. Iressa and a cytokine-expressing cellular vaccine such as GVAX® may find utility in the treatment of cancer.

### Delivery Of Cytokine-Expressincg Cellular Vaccines To The Patient

The present invention provides methods for cancer therapy, where a cytokine-expressing cellular vaccine and at least one additional cancer therapeutic agent or treatment are administered to a cancer patient. Desirably, the method effects a systemic immune response, i.e., a T-cell response and/or a B-cell response, to the cancer.

In a preferred aspect of the methods described herein, a cytokine-expressing cellular vaccine combination is administered to a cancer patient, wherein the cytokine-expressing cellular vaccine comprises mammalian, preferably human tumor cells, and the cells in the cytokine-expressing cellular vaccine are rendered proliferation incompetent, such as by irradiation. Administration of a cytokine-expressing cellular vaccine combination results in an enhanced immune response to the cancer as compared to the immune response to the same cancer following administration of the cytokine-expressing cellular vaccine or cancer therapeutic agent or treatment component of the combination alone. In other words, the combined administration of a cytokine-expressing cellular vaccine and at least one additional cancer therapeutic agent or treatment described above results in enhanced therapeutic efficacy as compared to administration of a cytokine-expressing cellular vaccine alone or administration of the cancer therapeutic agent(s) or treatment(s) alone.

The cytokine-expressing cellular vaccine combination may be administered by any suitable route. Preferably, the composition is administered subcutaneously or intratumorally. Local or systemic delivery can be accomplished by administration comprising administration of the combination into body cavities, by parenteral introduction, comprising intramuscular, intravenous, intraportal, intrahepatic, peritoneal, subcutaneous, or intradermal administration. In the event that the tumor is in the central nervous system, the composition is administered in the periphery to prime naïve T-cells in the draining lymph nodes. The activated tumor-specific T-cells are able to cross the blood/brain barrier to find their targets within the central nervous system.

In one exemplary preferred embodiment, the cytokine-expressing cellular vaccine is GVAX®, where the cytokine expressed is GM-CSF and the at least one additional, cancer therapeutic agent or treatment is selected from the group consisting of an antibody to CTLA4; an antibody to 4-1BB; interferon-alpha; a Cox-2 inhibitor; IL-2 or IL-18; docetaxel; an antibody to CD40 or membrane bound/soluble CD40 ligand; an antibody to OX40 or membrane bound/soluble OX40 ligand; a HSP (heat shock protein); a CpG dinucleotide; Iressa; Imiquimod; Juvaimmune; anti-epidermal growth factor R (EGF-R); anti-VEGF R, VEGF-trap or TRAIL.

As will be understood by those of skill in the art, the optimal treatment regimen will vary. As a result, it will be understood that the status of the cancer patient and the general health of the patient prior to, during, and following administration of a cytokine-expressing cellular vaccine combination, the patient will be evaluated in order to determine if the dose of each component and relative timing of administration should be optimized to enhance efficacy or additional cycles of administration are indicated. Such evaluation is typically carried out using tests employed by those of skill in the art to evaluate traditional cancer chemotherapy, as further described below in the section entitled "Monitoring Treatment".

Dependent upon the additional cancer therapeutic agent or treatment, the cytokine-expressing cellular vaccine is administered to the patient prior to, at the same time or following the administration of the additional cancer therapeutic agent or treatment. For example, in the case of an anti-CD40 antibody or CD40L, the GM-CSF-expressing cellular vaccine is administered to the patient prior to administration of an anti-CD40 antibody or CD40L.

### Delivery Of Chemotherapeutic Agents

In some aspects of the invention, the cytokine-expressing cellular vaccine combination comprises a chemotherapeutic agent. An important consideration in this aspect of the invention is effective delivery of the cancer therapeutic agent in a pharmaceutically acceptable carrier.

In accordance with this aspect of the invention, the choice of cancer therapeutic (i.e. chemotherapeutic) agent or agents and the corresponding route and timing of delivery takes advantage of one of more of: (i) established use in treatment of the particular type of cancer under treatment; (ii) the ability of the selected agent to result in an improved therapeutic outcome when administered in combination with the cytokine-expressing cellular vaccine; and (iii) delivery of the agent by a mode of administration effective to achieve sufficient localized exposure of the agent to cancer cells.

Typically, the cancer therapeutic agent is administered by a route and using a treatment regimen that has an established use in cancer therapy. As set forth above, the optimal route will vary with the cancer therapeutic agent. Local or systemic delivery can be accomplished by administration into body cavities, inhalation or insufflation of an aerosol, or by parenteral introduction, comprising intramuscular, intravenous, intraportal, intrahepatic, peritoneal, subcutaneous, or intradermal administration. However, preferred routes typically include slow intravenous infusion (IV drip), oral administration and local injection. In the event that the tumor is in the central nervous system, the composition must be administered into the periphery via any route. The formulations are easily administered in a variety of dosage forms such as injectable solutions, drug release capsules, implants or in combination with carriers such as liposomes or microcapsules.

Parenteral administration may be accomplished using a suitable buffered aqueous solution and the liquid diluent which has been prepared in isotonic form using saline or glucose. Such aqueous solutions are appropriate for intravenous, intramuscular, subcutaneous and intraperitoneal administration. (See, for example, "Remington's Pharmaceutical Sciences", 15th Edition, pages 1035-1038 and 1570-1580.) Sterile injectable solutions are prepared by incorporating the chemotherapeutic agent in the required amount of an appropriate solvent with various other ingredients included, followed by filter sterilization. Sterile powders for use in sterile injectable solutions may be prepared by vacuum drying or freeze drying techniques or other means to result in a powder of the active chemotherapeutic agent plus additional desired ingredients prepared from a previously sterile solution.

For example, when orally administered, the cancer therapeutic agent may be combined with an inert diluent or in an edible carrier, or enclosed in hard or soft shell gelatin capsules, compressed into tablets, incorporated directly into food, incorporated with excipients and used in the form of ingestible tablets, buccal tables, troches, capsules, elixirs, suspensions, syrups, wafers, and the like. The appropriate amount of cancer therapeutic agent is specific to the particular therapeutic agent and is generally known in the art.

Recommended dosages and dosage forms for a large number of cancer therapeutic agents have been established and can be obtained from conventional sources, such as the Physicians Desk Reference, published by Medical Economics Company, Inc., Oradell, N.J. Typically, the optimal route of delivery has been determined for known cancer therapeutic agents by well-established procedures and analysis, e.g., in clinical trials.

It will be understood that the invention contemplates treatment regimens that include the administration of one or more cancer therapeutic agents and administration of a cytokine-expressing cellular vaccine for therapy of cancer. Such a treatment regimen may be administered prior to, contemporaneously with, or subsequent to an additional cancer treatment, such as radiation therapy, further chemotherapy and/or immunotherapy.

The present invention provides the advantage that the dose of the one or more cancer therapeutic agents or treatments may be decreased when administered together with a cytokine-expressing cellular vaccine relative to treatment regimens that do not include cytokine-expressing cellular vaccine administration.

### Monitoring Treatment

One skilled in the art is aware of means to monitor the therapeutic outcome and/or the systemic immune response upon administering a combination treatment of the present invention. In particular, the therapeutic outcome can be assessed by monitoring attenuation of tumor growth and/or tumor regression and or the level of tumor specific markers. The attenuation of tumor growth or tumor regression in response to treatment can be monitored using several end-points known to those skilled in the art including, for instance, number of tumors, tumor mass or size, or reduction/prevention of metastasis.

All literature and patent references cited above are hereby expressly incorporated by reference herein.

### EXAMPLE 1

### Cytokine-Expressing Cellular Vaccines plus anti-CTLA-4 monoclonal antibody

In vivo studies were carried out using the B16F10 model to determine if an anti-CTLA-4 mAb (9D9), in combination with a cytokine-expressing cellular vaccine (such as GVAX®) can enhance anti-cancer efficacy. In one experiment, C57BI6 mice were challenged with 1x10⁵ B16F10 tumor cells on Day 0. One day later, mice were vaccinated with 3x10⁶ irradiated B16F10 or GM-CSF-secreting B16F10 (dB16gmtd) cells. One and three days after vaccination, mice were injected with 150µg or 100µg of anti-CTLA-4 antibody (9D9 or 9H10) respectively. Mice received a second vaccination of 3x10⁶ irradiated B16F10 or GM-CSF-secreting B16F10 cells two days after the anti-CTLA-4 treatment (Fig. 2A). Mice were then monitored for the development of subcutaneous tumors. Two anti-CTLA-4 mAbs, 9D9 (murine anti-CTLA4) and 9H10 (hamster anti-CTLA4) were tested in the B16 melanoma tumor model (Fig. 2A) and shown to have similar efficacy. Due to immunological reaction to the hamster anti-CTLA4 antibody, the murine version was used in subsequent studies.

The 9D9 anti-CTLA-4 mAb was tested in the B16 melanoma tumor model with and without vaccination with a cytokine-expressing cellular vaccine (GM-CSF-secreting B16F10 tumor cells; B16-GM). The results show that the 9D9 anti-CTLA-4 mAb was ineffective as a monotherapy in protecting C57BI/6 mice from B16F10 tumor challenge. In contrast, 80% of the mice treated with B16-GM plus 9D9 were protected, demonstrating that the 9D9 anti-CTLA4 mAb is biologically active and further supports the concept that anti-CTLA-4 and GVAX® vaccines are an effective anti-cancer combination therapy (Fig. 2B).

### Immune Response Following Repeated Vaccination With a GM-CSF-Secreting Vaccine Combined With Treatment With An α-CTLA-4 Antibody

Another study in mice was used to evaluate the immune response to repeated vaccination with a GM-CSF-secreting vaccine combined with treatment with the α-CTLA-4 9D9 monoclonal antibody. In this study, non-tumor-bearing C57BI/6 male mice received five vaccinations with 3 x 10⁶ irradiated mGM-CSF-secreting tumor cells derived from a spontaneously occurring murine prostate adenocarcinoma, which secreted 100 ng GM-CSF/10⁶ cells/24 hr or non-transduced murine prostate adenocarcinoma cells on a biweekly schedule, Selected treatment groups received IP injections of anti-CTLA-4 9D9 or a mouse IgG2b isotype antibody control 1 and 4 days (100 ug each) after vaccination numbers 1, 3 and 5. Mice were necropsied at weeks 2, 4, 6, 8,10 and 13 for assessment of immunological and safety endpoints. Two weeks after the last vaccination, spleen cells were analyzed by flow cytometry for prostate tumor-specific antigen (PTSA)-specific intracellular IFN-gamma expression.

Immune responses were measured as increases in tumor antigen-specific T-cells. Such T-cells can be identified by the induction of intracellular IFN-gamma expression following stimulation with a tumor-specific peptide bound to MHC I. A prostate tumor-specific antigen that is both a murine prostate adenocarcinoma cell-specific epitope and shared with human prostate cancer. Intracellular cytokine staining (ICS) may be used to monitor tumor-specific T-cell responses and to identify very low frequencies of antigen-specific T-cells. Because ICS is performed on freshly isolated lymphocytes within 5 hours of removal, unlike the cytolytic assay, which often requires 5-7 days of in vitro stimulation, it can be used to estimate the frequency of tumor antigen-specific T-cells in vivo. This provides a means to compare the potency of different tumor vaccine strategies. In this study, ICS was utilized to monitor T-cell responses to the prostate tumor-specific antigen described above after vaccination with the murine prostate adenocarcinoma cell-GM vaccine with and without CTLA-4 9D9 co-treatment. The results are shown in Fig. 3. The number above each bar represents the fold increase of PTSA-specific IFN-gamma⁺CD8⁺ T-cells from each treatment group compared to naïve mice. NP-1 is a non-specific peptide control.

A 10-16 fold increase in the frequency of antigen specific T cells was detected in mice that received multiple vaccinations with a GM-CSF secreting tumor cell in the absence of anti-CTLA-4 when compared to non-immunized controls. Similarly, a single injection of anti-CTLA-4 after the first vaccination only, increased the prostate tumor antigen (PTSA)-specific T cell response 10 fold above naïve mice. However the most striking responses were observed in mice that received anti-CTLA-4 injections after the first, third, and fifth vaccine. In this group, a 36-fold increase in PTSA-specific T cell response was detected. These data demonstrate that multiple vaccinations with a GM-CSF-secreting tumor cell vaccine combined with repeated anti-CTLA-4 injections (i.e. CTLA-4 blockade) induce a potent tumor-specific T-cell response. The synergism of the combination of GM-CSF-secreting vaccines and anti-CTLA-4 antibodies in the B16 melanoma model suggest the potential clinical benefit of the combination.

A further suggestion as to the potential utility if the combination of GM-CSF-secreting vaccines and anti-CTLA-4 antibodies in eliciting an anti-tumor immune response is the production of effector cytokines such as TNF-alpha, IL-2, and IFN-gamma upon restimulation in vitro. Release of such cytokines is often used as a surrogate marker for monitoring tumor-specific immune responses following immunotherapeutic strategies designed to induce anti-tumor immunity. When cytokine levels were measured in supernatants from spleen cells restimulated in vitro for 48 hours with irradiated GM-CSF-secreting tumor cells the production of TNF-alpha, IFN-gamma, IL-5 and IL-2 was detected by week two (data not shown).

### EXAMPLE 2

### Cytokine-Expressing Cellular Vaccine (GVAX®) + anti-41BB mAb

In vivo studies were carried out using the B16F10 model to determine if an anti-41BB monoclonal antibody in combination with a cytokine-expressing cellular vaccine (GVAX®) can enhance anti-cancer efficacy.

On day 0 mice were challenged with 1x10⁵ live B16F10 (by sub-cu (SC) injection at a dorsal site in 0.5ml volume). On day 3, mice were vaccinated with 1x10⁶ irradiated vaccine cells (as indicated in Table 2, below), followed by injection of 150ug of anti-41BB mAb IP on day 6 (IP in 0.5ml volume).

**Table 2. B16F10 Tumor Model Treatment Groups For Combinations Comprising anti-41BB Antibody**

| Group # | # mice | Challenge | Vaccination | Treatment |
|---|---|---|---|---|
| 100 A-L | 12 | B16F10 | HBSS | None |
| 200 A-L | 12 | B16F10 | B16F10 | None |
| 300 A-L | 12 | B16F10 | GVAX® | None |
| 400 A-L | 12 | B16F10 | GVAX® | 41BB |

Mice were assessed daily for any obvious abnormality and if subcutaneous tumors reached 15-20mm-diameter in size or started to ulcerate through the skin animals were euthanized. On day 21, spleens and blood were collected and used for analysis of anti-tumor CTL, cytokine release (ELISA) and intracellular FACS.

The results indicate that the combined administration of GVAX® and anti-4-1BB antibody enhanced both the number of tumor free mice (Fig. 4A) and survival (Fig. 4B) relative to administration of GVAX® alone, for up to 80 days post-challenge.

### EXAMPLE 3

### Cytokine-Expressing Cellular Vaccine (GVAX®) + Interferon-Alloha

*In vitro* studies showed that IFN-alpha inhibits tumor cell proliferation and induces apoptosis in a dose dependent manner. The results shown in Fig. 5A are derived from a study where murine tumor cell lines were co-cultured with recombinant murine IFN-alpha (rmIFN-a) in 96 well plates in triplicate for 72 hr. Cells were pulsed with ³H thymidine and proliferation was measured by radioactivity uptake. Individual tumor cell lines expressed different levels of inhibition when co-cultured with rmIFN-a. The anti-proliferative effect of IFN-alpha was most evident with the B16F10 cell line ' relative to the other cell lines tested. The results shown in Fig. 5B are derived from a study where after the murine tumor cell lines were co-cultured with rmIFN-alpha in 96 well plates in triplicate for 72 hr, the cells were trypsinized, stained with Annexin-V and 7-AAD and analyzed by flow cytometry for apoptosis. Individual tumor cell lines expressed different levels of apoptosis when co-cultured with rm IFN-alpha. CT26 was the most sensitive cell line tested to the pro-apoptotic effect of IFN-alpha,

The anti-proliferative effects were observed in a variety of tumors, including melanoma (B16F10), renal cell carcinoma (renca), colon carcinama (CT26), breast carcinoma (4T1), lung cancer (LLC) and fibrosarcoma (3T3). Incubation with 5000 U/ml of IFN-alpha inhibited proliferation between 20-40% in most tumors. However, the same dose of IFN-alpha inhibited B16F10 melanoma cells by greater than 80%. Pro-apoptotic activity was also observed in all tumor cells tested. The increase in apoptosis ranged between 1.5-2.5 fold above controls in all tumors with the exception of CT26 in which there was a 5-fold increase in apoptosis. IFN-alpha is provided in the form of the protein (recombinant mouse IFN-Ipha: R + D Systems), or as an IFN-alpha producing cell line prepared by transduction of cells with a vector comprising the coding sequence for IFN-alpha. Exemplary interferon-alpha sequences for use in practicing the invention may be found for example at GenBank Accession Nos. NM1FNA1, NM1FNA2, expressly incorporated by reference herein.

In vivo studies were also carried out to determine if interferon-afpha in combination with a cytokine-expressing cellular vaccine (GVAX®) can enhance anti-cancer efficacy. A schematic depiction of an exemplary study is provided in Fig. 6. In one exemplary protocol, C57B16 mice were challenged with 1x10⁵ B16F10 tumor cells. Three days later, mice were vaccinated with 1x10⁶ irradiated B16F10 or GM-CSF-secreting B16F10 (B16.GM) cells. Four days after the vaccine, IFN-alpha administration was initiated. IFN-alpha (2500 units/day; twice/week) was injected subcutaneously at the site of tumor challenge (Fig. 7A) or the vaccination site (Fig. 7B). Tumor establishment was determined by palpation every 3-4 days. Mice with necrotic tumors or tumor size of 150mm² were sacrificed. Data shown is from 10 mice/study expressed as percentage of survival at the indicated time points. Survival efficacy of B16.GM was 40% and B16.GM + IFN-alpha was 40% above B16.GM monotherapy with the significance of p=0.08. Survival efficacy of B16.GM was 10% and B16.GM + IFN-alpha was 40% above B16.GM monotherapy with the significance of (p=0,08).

In summary, these data suggest that local IFN-alpha treatment at close proximity to the resected tumor given following resection of the primary tumor is effective to keep the tumor dormant. Furthermore, IFN-alpha may be administered in conjunction with the vaccine at the vaccine site. Therefore, IFN-alpha combined with a GM-CSF secreting whole cell vaccine results in increased survival in the B16F10 tumor model, and represents a new clinical combination for tumor immunotherapy.

### EXAMPLE 4

### Cytokine-Expressing Cellular Vaccine (GVAX®) + docetaxel (Taxotere™)

The combination of docetaxel and a GM-CSF-secreting B16F10 tumor cell (B16-GM) vaccine was evaluated in the B16 melanoma model. Three IV injections of docetaxel (for a total dose 18 mg/kg) on days 5, 9, and 13 were ineffective as a monotherapy at inhibiting B16 tumor growth, with a median survival of 24 days (Figs. 8A and 8B), Mice vaccinated with B16-GM three days after tumor challenge also had a median survival of 24 days. In contrast, the combination of docetaxel and B16-GM using the same treatment regimen, significantly delayed tumor growth and increased the median survival to 45 days (Figs. 8C and 8D). Administration of docetaxel given between bi-weekly B16-GM vaccinations increased the median survival of B16 tumor-bearing mice from 31 days to 52 days when compared multiple B16-GM vaccinations alone. These effects were also observed using a multiple B16-GM vaccine plus multiple docetaxel regimen. (Table 3).

**Table 3. Effect Of Multiple B16-GM Vaccine Plus Multiple Docetaxel Regimen On Survival**

| Treatment | Median Survival (days) |
|---|---|
| HBSS | 24 |
| Taxotere | 24 |
| GVAX® 1x | 24 |
| GVAX® (3x) | 31 |
| GVAX® 1x + Tax 1x | 45 |
| GVAX® 3x + Tax 3x | 52 |

Furthermore, by using B16 tumor cells that express membrane-bound ovalbumin, where ovalbumin is used as a model tumor antigen that permits tracking of antigen-specific T-cells, it was demonstrated that administration of docetaxel prolongs the survival of ovalbumin-specific TCR transgenic T cells (OT-1; data not shown). These data suggest that administration of docetaxel can enhance the potency of a GM-CSF secreting tumor cell vaccine by enhancing the survival of tumor-specific effector T cells.

### EXAMPLE 5

### Cytokine-Expressing Cellular Vaccine (GVAX®) + a COX-2 inhibitor

The combination of the COX-2 inhibitor, Celecoxib (CELEBREX ®), has been evaluated in combination with B16-GM in the 616F10 model to test whether a cytokine-expressing cellular vaccine plus Celebrex affords greater protection than vaccination with either agent alone. Preliminary results show that Celebrex alone had no effect on B16F10 tumor size and overall survival as a single agent. However, when combined with a GM-CSF-expressing cellular vaccine, a significant increase in the number of surviving and tumor free animals was observed. The results show that Celebrex must be administered at least 3 days before vaccination with the cytokine-expressing cellular vaccine in order for efficacy (Figs. 9A and B). If Celebrex is administered on the day of vaccine, added efficacy was observed relative to that observed when the cytokine-expressing cellular vaccine is administered alone. In further studies, we have also demonstrated that as a monotherapy Celebrex does have a significant effect on CT26 tumor size and overall survival of BALB/c mice.

### EXAMPLE 6

### Cytokine-Expressing Cellular Vaccine (GVAX®) + CD40L

A variety of in vivo studies have been carried out to evaluate the potential of cytokine-expressing cellular vaccine combinations which include anti-CD40 and CD40L, as well as the parameters for optimizing the efficacy of such combinations. Factors to be considered in designing clinical studies include but are not limited to, relative timing of administration of the components of the combination, dose of each component, route and frequency of administration. In vivo studies were carried out to determine if anti-CD40 or CD40L in combination with a cytokine-expressing cellular vaccine (GVAX®) can enhance anti-cancer efficacy of a cytokine-expressing cellular vaccine alone (Fig. 10). In one exemplary experiment, Ad-NULL(E-1 deleted adenovirus expressing no transgene), Ad-GVAX® (Ad-GM-CSF) and Ad-CD40L vaccines were prepared and tumor cells transduced using standard procedures, such as described in WO 00/72686, expressly incorporated by reference herein. In carrying out such experiments either separate populations of cells were transduced with each of Ad-GM-CSF and Ad-CD40L or the same population of cells was transduced with both vectors. When separate populations of cells are transduced with the individual vectors, the cell populations may be mixed and administered at the same time or administered at different times as described below. In one experiment, on day 0, mice in Groups 1-11 were challenged with 1 x 10⁵ live B16F10 mouse melanoma cells, (SC dorsal site in 0,5ml volume). On day 3 after challenge, mice were vaccinated ventrally with 1 x 10⁶ B16-GM, followed by administration of further irradiated vaccine cells on day 3 (Group 5), day 4 (Groups 6 and 7), day 5 (Groups 8 and 9) and day 7 (Groups 10 and 11), as shown below in Table 4. Mice were monitored for tumor burden every 3-4 days for 3 months (n=10) and sacrificed on day 18, with spleens and blood collected for immunological assays. The results shown in Fig. 11 demonstrate that B16F10 cells transduced by an adenovirus vector encoding CD40 express CD40L, and when administered in combination with B16-GM, significantly increase survival of mice challenged with B16F10 melanoma, in particular when Ad-CD40L-transduced cells were administered 4 days after vaccination. When CD40L was administered the same day as B16-GM, the disease was exacerbated.

**Table 4. B16F10 Tumor Model Treatment Groups For Combinations CD40 Ligand**

| Group | # Mice | Vaccination |
|---|---|---|
| 1 | 12 | HBSS |
| 2 | 12 | Ad-Null (100 MOI) |
| 3 | 12 | Ad-GVAX® (100 MOI) + Ad-Null (100 MOI) (1:1) |
| 4 | 12 | Ad-CD40L (100 MOI) + Ad-Null (100 MOI) (1:1) |
| 5 | 12 | Ad-GVAX® (100 MOI) + Ad-CD40L (100 MOI) (1:1) Day of GVAX® |
| 6 | 12 | Ad-GVAX® (100 MOI) + Ad-CD40L (100 MOI) (1:1) Day 1 post GVAX® |
| 7 | 12 | Ad-GVAX® (100 MOI) + Ad-Null (100 MOI) (1:1) Day 1 post GVAX® |
| 8 | 12 | AD-GVAX® (100 MOI) + Ad-CD40L (100 MOI) (1:1) Day 2 post GVAX® |
| 9 | 12 | Ad-GVAX® (100 MOI) + Ad-Null (100 MOI) (1:1) Day 2 post GVAX® |
| 10 | 12 | Ad-GVAX®) (100 MOI) + Ad-CD40L (100 MOI) (1:1) Day 4 post GVAX® |
| 11 | 12 | Ad-GVAX® (100 MOI) + Ad-Null (100 MOI) (1:1) Day 4 post GVA)A) |

| | | |
|---|---|---|
| MOI stands for multiplicity of infection which represents the number of infectious viral particles to tumor cells. | | |

To further demonstrate the efficacy of B16-CM anti-CD40 combinations, animals were treated with an anti-CD40 monoclonal antibody administered at different times post vaccination. In this experiment, on day 0 mice were challenged with 1 x 10⁵ live B16F10 mouse melanoma cells, (SC dorsal site in 0.5ml volume). On day 3 after challenge, mice were vaccinated ventrally with 1 x 10⁶ B16-GM. Anti-CD40 antibody was administered on days 0 and 2 or days 2 and 4 (Fig. 11B); days 4 and 6, days 7 and 9, or and days 10 and 12 (Fig. 11C). Mice were monitored for tumor burden every 3-4 days for 3 months (n=10) and sacrificed on day 18, with spleens and blood collected for immunological assays.

The results demonstrate that the combination of either membrane bound CD40L or monoclonal anti-CD40 antibody and B16-GM vaccine significantly enhances the efficacy of B16F10 vaccine expressing GM-CSF in the B16-GM melanoma model. The results from B16 melanoma model studies further show that the timing of CD40L and anti-CD40 administration is critical to efficacy (Figs. 11A -11C).

### EXAMPLE 7

### Cytokine-Expressing Cellular Vaccine (GVAX®) + OX40/OX40L

The efficacy of a cytokine-expressing cellular vaccine (e.g., GVAX®) and anti-OX40 or membrane bound/soluble OX40L is evaluated using the B16F10 melanoma tumor model using the exemplary study design shown in Table 5, below.

**Table 5. B16F10 Tumor Model Treatment Groups For Combinations Comprising Anti-OX40 or OX40L:Ig Fusion Protein**

| GROUP | TREATMENT |
|---|---|
| 1 | HBSS only (negative control) |
| 2 | Irradiated BI6FIO/HBSS (control) |
| 3 | GVAX®/HBSS (GVAX® as monotherapy) |
| 4 | Anfi-OX40 or OX40L:Ig fusion protein only as monotherapy |
| 5 | Irradiated B16F10/OX40 stimulation (Combination of anti-OX40 plus relevant |
| 6 | Irradiated GVAX®/OX40 (Combination GVAX® and OX40 test group) |

In optimizing the treatment protocol, a number of variables are important, as outlined in Table 6, below.

**Table 6. Combination therapy with dB16-retro GM-CSF secreting cells and OX40.**

| Combination | Variable | # per group | # of groups | Total/ Experiment | # of Expts. | Total # of Mice |
|---|---|---|---|---|---|---|
| Anti-OX40 | Anti-OX40 dosed at the same time as GVAX® | 12 | 6-10 | 72-120 | 2-3 | 144-360 |
| Anti-OX40 | Timing relative to GVAX® | 12 | 6-10 | 72-120 | 2-3 | 144-360 |
| Anti-OX40 | Single vs multiple GVAX® plus Anti-OX40 | 12 | 6-10 | 72-120 | 2-3 | 144-360 |
| OX40L Expression | Soluble vs membrane bound OX40L on vaccine cells | 12 | 6-10 | 72-120 | 2-3 | 144-360 |
| Anti-OX40 | Different tumor Models (CT26, 4TI, etc) | 12 | 5 | 60 | 2-3 | 120-180 |

Based on published literature and various experimental results, OX40-mediated protection ranges from 20-60% depending on the inherent immunogenicity of the tumor (Weinberg, A. et al., J. Immunol., 164: 2160-2169, 2000). The degree of anti-tumor protection afforded by the combination of GVAX® and OX40 is dependent upon several factors such as timing relative to GVAX®, the route of administration and the relative, dose of anti-OX40 or OX40L:Ig fusion protein and GVAX®.

### EXAMPLE 8

### Cytokine-Expressing Cellular Vaccine (GVAX®) + Hsp70

The efficacy of a cytokine-expressing cellular vaccine (e.g., GVAX®) and Hsp70 is evaluated using the B16F10 melanoma tumor model, An exemplary study design follows:

**Table 7. B16F10 Tumor Model Treatment Groups For Combinations Comprising hsp70**

| GROUP | TREATMENT |
|---|---|
| 1 | HBSS only (negative control) |
| 2 | Irradiated B16FIO/HBSS (control) |
| 3 | GVAX®/HBSS (GVAX® as monotherapy) |
| 4 | Irradiated B16F10/hsp70 (hsp 70 as monotherapy) |
| 5 | Irradiated GVAX®/hsp70 (Combination GVAX® /hsp70) |

In optimizing the treatment protocol, a number of variables are important, such as outlined in Table 8, below.

**Table 8. Combination therapy with dB16-retro GM-CSF secreting: cells and Hsp70**

| **Combination** | **Variable** | **# per group** | **# of groups** | **Total/ Expt.** | **# of Expts.** | **Total # of Mice** |
|---|---|---|---|---|---|---|
| hsp70 | Dose of hsp70 at the same time as GVAX® | 12 | 6-10 | 72-120 | 2-3 | 144-360 |
| hsp70 | Timing of hsp70 relative to GVAX® | 12 | 6-10 | 72-120 | 2-3 | 144-360 |
| hsp70 | Single vs multiple doses of GVAX® plus hsp70 | 12 | 6-10 | 72-120 | 2-3 | 144-360 |

The degree of anti-tumor protection afforded by the combination of GVAX® and hsp70 will be dependent upon several factors such as timing of HSP administration relative to that of GVAX®, the route of administration and the relative dose of GVAX® versus the dose of hsp70.

### Embodiments

1. An improved method for cancer therapy, comprising:
   administering the combination of a cytokine-expressing cellular vaccine and at least one additional cancer therapeutic agent selected from the group consisting of an anti-CTLA4 antibody, an anti-4-IBB antibody, interferon-alpha, docetaxel, paclitaxel, a COX-2 inhibitor, an anti- CD40 antibody or CD40 ligand, an anti-OX40 antibody or OX-40 ligand and a heat shock protein (HSP), to a subject with cancer, wherein administration of the combination to the subject results in enhanced therapeutic efficacy relative to administration of the cytokine-expressing cellular vaccine or the at least one additional cancer therapeutic agent alone.
2. The method of item 1, wherein the cytokine-expressing cellular vaccine expresses GM-CSF.
3. The method of item 2, wherein the cells of said cytokine-expressing cellular vaccine are autologous to the subject.
4. The method of item 2, wherein the cells of said cytokine-expressing cellular vaccine are allogeneic to the subject.
5. The method of item 2, wherein the cells of said cytokine-expressing cellular vaccine cells are bystander cells.
6. The method of item 2, wherein the cells of the cytokine-expressing cellular vaccine are rendered proliferation-incompetent by irradiation.
7. The method of item 2, wherein the mammal is a human.
8. The method of item 2, wherein the cancer is a prostate cancer.
9. The method of item 2, wherein the cancer is a non-small cell lung carcinoma.
10. The method of item 4, wherein the allogeneic cells are a tumor cell line selected from the group consisting of a prostate tumor line, a non-small cell lung carcinoma line and a pancreatic cancer line.
11. The method of item 2, wherein said at least one additional cancer therapeutic agent includes an anti-CTLA4 antibody.
12. The method of item 2, wherein said at least one additional cancer therapeutic includes an anti-4-166 antibody.
13. The method of item 2, wherein said at least one additional cancer therapeutic agent includes interferon-alpha.
14. The method of item 2, wherein said at least one additional cancer therapeutic agent includes docetaxel or paclitaxel.
15. The method of item 14, wherein said at least one additional cancer therapeutic agent includes docetaxel.
16. The method of item 2, wherein said at least one additional cancer therapeutic agent includes a COX-2 inhibitor.
17. The method of item 16, wherein said COX-2 inhibitor is Celecoxib.
18. The method of item 2, wherein said at least one additional cancer therapeutic agent includes an anti-CD40 antibody or CD40 ligand.
19. The method of item 2, wherein said at least one additional cancer therapeutic agent is expressed by a cell and the cell is an autologous, allogeneic or a bystander cell.
20. The method of item 19, wherein the autologous, allogeneic or a bystander cell is rendered proliferation-incompetent by irradiation.
21. The method of item 20, wherein the autologous, allogeneic or a bystander cell expresses interferon-alpha.
22. The method of item 20, wherein the autologous, allogeneic or a bystander cell expresses CD40 ligand.
23. The method of item 2, wherein said cytokine-expressing cellular vaccine is administered subcutaneously.
24. The method of item 2, wherein said cytokine-expressing cellular vaccine is administered intratumorally.
25. The method of item 16, wherein said COX-2 inhibitor is administered before the GM-CSF-expressing cellular vaccine.
26. The method of item 18, wherein said anti-CD40 antibody is administered after the GM-CSF-expressing cellular vaccine.
27. The method of item 18, wherein said CD40 ligand is administered after the GM-CSF-expressing cellular vaccine.
28. An improved composition for cancer therapy, comprising:
   a GM-CSF expressing cellular vaccine and at least one additional cancer therapeutic agent selected from the group consisting of an anti-CTLA4 antibody, an anti-4-1BB antibody, interferon-alpha, docetaxel, Celecoxib, an anti- CD40 antibody and CD40 ligand for administration to a subject with cancer, wherein administration of the combination results in enhanced therapeutic efficacy relative to administration of the GM-CSF expressing cellular vaccine or the at least one additional cancer therapeutic agent alone.
29. The composition of item 28, wherein the cells of said cytokine-expressing, cellular vaccine are autologous to the subject.
30. The composition of item 28, wherein the cells of said cytokine-expressing cellular vaccine are allogeneic to the subject.
31. The composition of item 28, wherein the cells of said cytokine-expressing cellular vaccine cells are bystander cells.
32. The composition of item 28, wherein the cells of said cytokine-expressing cellular vaccine are rendered proliferation-incompetent by irradiation.
33. The composition of item 30, wherein said allogeneic cells are a tumor cell line selected from the group consisting of a prostate tumor line, a non-small cell lung carcinoma line and a pancreatic cancer line.
34. The composition of item 28, wherein said at least one additional cancer therapeutic agent is an anti-CTLA4 antibody.
35. The composition of item 28, wherein said at least one additional cancer therapeutic is an anti-4-1BB antibody.
36. The composition of item 28, wherein said at least one additional cancer therapeutic agent is interferon-alpha.
37. The composition of item 28, wherein said at least one additional cancer therapeutic agent is docetaxel.
38. The composition of item 28, wherein said at least one additional cancer therapeutic agent is Celecoxib.
39. The composition of item 28, wherein said at least one additional cancer therapeutic agent is an anti-CD40 antibody or CD40 ligand.
40. The composition of item 28, wherein said at least one additional cancer therapeutic agent is expressed by a cell and the cell is autologous, allogeneic or a bystander cell.
41. The composition of item 40, wherein the autologous, allogeneic or bystander cell is rendered proliferation-incompetent by irradiation.
42. The composition of item 41, wherein the autologous, allogeneic or a bystander cell expresses interferon-alpha.
43. The composition of item 41, wherein the he autologous, allogeneic or a bystander cell expresses CD40 ligand.

## Claims

1. Use of a combination of a cytokine-expressing cellular vaccine and interferon-alpha for the manufacture of a medicament for treating cancer in a mammal.

2. The use of claim 1, wherein the cytokine-expressing cellular vaccine expresses GM-CSF.

3. The use according to claim 1 or 2, wherein the cells of said cytokine-expressing cellular vaccine are autologous to the subject.

4. The use according to claim 1 or 2, wherein the cells of said cytokine-expressing cellular vaccine are allogeneic to the subject.

5. The use according to claim 1 or 2, wherein the cells of said cytokine-expressing cellular vaccine cells are bystander cells.

6. The use according to any one of Claims 1 through 5, wherein the cells of the cytokine-expressing cellular vaccine are rendered proliferation-incompetent by irradiation.

7. The use according to any one of Claims 1 through 5, wherein the mammal is a human.

8. The use according to any one of Claims 1 through 5, wherein the cancer is a hematopoietic tumor.

9. The use of any one of claims 4, 6 or 7, wherein the allogeneic cells are a tumor cell line selected from the group consisting of a hematopoietic tumor line, a lymphoid cell line and a myeloid cell line.

10. The use according to any one of Claims 1 through 9, wherein said interferon-alpha is expressed by a cell and the cell is an autologous, allogeneic or a bystander cell.

11. The use of claim 10, wherein the autologous, allogeneic or a bystander cell is rendered proliferation-incompetent by irradiation.

12. The use of claim 10, wherein said interferon-alpha is expressed by the cytokine-expressing cellular vaccine.
